# EUROPÄISCHE PATENTSCHRIFT

(11) **EP 3 083 943 B1**
(45) Veröffentlichungstag und Bekanntmachung des Hinweises auf die Patenterteilung: **29.05.2019**
(21) Anmeldenummer: 14812204.7
(22) Anmeldetag: 10.12.2014
(51) Int. Cl.: C12N 5/077

(54) **VERFAHREN ZUR ERZEUGUNG VON SINUSKNOTENZELLEN ("HERZ-SCHRITTMACHERZELLEN") AUS STAMMZELLEN UND VERWENDUNG DER ERZEUGTEN SINUSKNOTENZELLEN**
METHOD FOR PRODUCING SINOATRIAL NODE CELLS (PACEMAKER CELLS) FROM STEM CELLS, AND USE OF THE PRODUCED SINOATRIAL NODE CELLS
PROCÉDÉ DE GÉNÉRATION DE CELLULES DE NOEUDS SINUSAUX (CELLULES DE STIMULATEURS CARDIAQUES) À PARTIR DE CELLULES SOUCHES ET UTILISATION DES CELLULES DE NOEUDS SINUSAUX GÉNÉRÉES

(30) Priorität: 20.12.2013 DE 102013114671
(43) Veröffentlichungstag der Anmeldung: 26.10.2016
(73) Patentinhaber: Universität Rostock, 18055 Rostock (DE)
(72) Erfinder: DAVID, Robert, 86415 Mering (DE); JUNG, Julia, 18109 Rostock (DE)
(74) Vertreter: Herzog, Fiesser & Partner Patentanwälte PartG mbB
(86) Internationale Anmeldenummer: PCT/EP2014/077215
(87) Internationale Veröffentlichungsnummer: WO 2015/091157

(56) Entgegenhaltungen:
- EP-A1- 2 484 756
- WO-A1-2013/070952
- A. SCAVONE ET AL: "Embryonic Stem Cell-Derived CD166+ Precursors Develop Into Fully Functional Sinoatrial-Like Cells", CIRCULATION RESEARCH, Bd. 113, Nr. 4, 2. August 2013 (2013-08-02), Seiten 389-398, XP055163060, ISSN: 0009-7330, DOI: 10.1161/CIRCRESAHA.113.301283
- M. L. BAKKER ET AL: "T-box transcription factor TBX3 reprogrammes mature cardiac myocytes into pacemaker-like cells", CARDIOVASCULAR RESEARCH, Bd. 94, Nr. 3, 1. Juni 2012 (2012-06-01), Seiten 439-449, XP055163087, ISSN: 0008-6363, DOI: 10.1093/cvr/cvs120
- G. KENSAH ET AL: "Murine and human pluripotent stem cell-derived cardiac bodies form contractile myocardial tissue in vitro", EUROPEAN HEART JOURNAL, Bd. 34, Nr. 15, 26. Oktober 2012 (2012-10-26), Seiten 1134-1146, XP055163775, ISSN: 0195-668X, DOI: 10.1093/eurheartj/ehs349
- OTSUJI T G ET AL: "Progressive maturation in contracting cardiomyocytes derived from human embryonic stem cells: Qualitative effects on electrophysiological responses to drugs", STEM CELL RESEARCH, ELSEVIER, NL, Bd. 4, Nr. 3, 1. Mai 2010 (2010-05-01), Seiten 201-213, XP027054730, ISSN: 1873-5061 [gefunden am 2010-02-06]
- KUMI MORIKAWA ET AL: "Identification, Isolation and Characterization of HCN4-Positive Pacemaking Cells Derived from Murine Embryonic Stem Cells during Cardiac Differentiation", PACING AND CLINICAL ELECTROPHYSIOLOGY, Bd. 33, Nr. 3, 1. März 2010 (2010-03-01), Seiten 290-303, XP55099647, ISSN: 0147-8389, DOI: 10.1111/j.1540-8159.2009.02614.x
- BARBUTI A ET AL: "Mesoangioblasts from ventricular vessels can differentiate in vitro into cardiac myocytes with sinoatrial-like properties", JOURNAL OF MOLECULAR AND CELLULAR CARDIOLOGY, ACADEMIC PRESS, GB, Bd. 48, Nr. 2, 1. Februar 2010 (2010-02-01), Seiten 415-423, XP026862693, ISSN: 0022-2828 [gefunden am 2009-10-22]
- LAURINE MARGER ET AL: "Pacemaker activity and ionic currents in mouse atrioventricular node cells", CHANNELS, Bd. 5, Nr. 3, 1. Mai 2011 (2011-05-01), Seiten 241-250, XP055163903, ISSN: 1933-6950, DOI: 10.4161/chan.5.3.15264
- KLUG M ET AL: "Genetically selected cardiomyocytes from differentiating embronic stem cells form stable intracardiac grafts", JOURNAL OF CLINICAL INVESTIGATION, AMERICAN SOCIETY FOR CLINICAL INVESTIGATION, US, Bd. 98, Nr. 1, 1. Juli 1996 (1996-07-01), Seiten 216-224, XP002180457, ISSN: 0021-9738, DOI: 10.1172/JCI118769
- JULIA JEANNINE JUNG ET AL: "Programming and Isolation of Highly Pure Physiologically and Pharmacologically Functional Sinus-Nodal Bodies from Pluripotent Stem Cells", STEM CELL REPORTS, Bd. 2, Nr. 5, 1. Mai 2014 (2014-05-01), Seiten 592-605, XP055162914, ISSN: 2213-6711, DOI: 10.1016/j.stemcr.2014.03.006
- Susann Hartung ET AL: "Directing Cardiomyogenic Differentiation of Human Pluripotent Stem Cells by Plasmid-Based Transient Overexpression of Cardiac Transcription Factors", STEM CELLS AND DEVELOPMENT, vol. 22, no. 7, 1 April 2013 (2013-04-01), pages 1112-1125, XP055385160, NL ISSN: 1547-3287, DOI: 10.1089/scd.2012.0351
- DAVID R ET AL: "Forward programming of pluripotent stem cells towards distinct cardiovascular cell types", CARDIOVASCULAR RESE, OXFORD UNIVERSITY PRESS, GB, vol. 84, no. 2, 1 November 2009 (2009-11-01), pages 263-272, XP008121282, ISSN: 0008-6363, DOI: 10.1093/CVR/CVP211 [retrieved on 2009-06-29]
- HALBACH M ET AL: "1603: Long-term persistence, functional integration and electrophysiological properties of transplanted cardiomyocytes derived from pluripotent stem cells", EUROPEAN HEART JOURNAL; ESC CONGRESS 2013, OXFORD UNIVERSITY PRESS, GB; AMSTERDAM, NETHERLANDS, vol. 34, no. Suppl. 1, 1 August 2013 (2013-08-01), page 315, XP009194774, ISSN: 0195-668X, DOI: 10.1093/EURHEARTJ/EHT308.1603

## Beschreibung

Der Begriff des "Sick-Sinus-Syndroms" wird als Sammelbegriff für die Umschreibung einer Reihe von Erkrankungen verwendet, die durch eine gestörte Funktion des Sinusknotens, dem Schrittmacher des Herzens, hervorgerufen werden. Er ist strukturell aus spezialisierten Kardiomyozyten aufgebaut, die durch das autonome Nervensystem innerviert werden. Zu den Erkrankungen gehören die pathologische symptomatische Sinusbradykardie, SA-Block (SA = Sinu-Atrial), Sinusarrest sowie das Tachykardie-Bradykardie-Syndrom.

Das Sick-Sinus-Syndrom wird häufig von allgemeinen Herzerkrankungen wie ischämischer Herzerkrankung, Kardiomyopathien oder Myokarditis begleitet. Diese führen entweder zu einer gestörten Erregungsbildung innerhalb des Sinusknotens oder zu einer gestörten Übertragung der elektrischen Impulse von dem Sinusknoten zum Vorhof (letzteres wird als "sinuatriale Leitungsstörung" bezeichnet). Momentan stützen sich die Therapiemaßnahmen für das "Sick-Sinus-Syndrom" auf die Implantation künstlicher Schrittmacher, die äußerst kostenintensiv sind und keine Sensitivität für eine hormonelle Stimulation aufweisen. Dazu kommt, dass Gefährdungen durch Infektionen und vorzeitige Batterieentladung zu maßgeblichen Einschränkungen führen. Somit sind Patienten mit einem implantierten Schrittmacher in ihrer verbleibenden Lebenszeit generell einem hohen Risiko ernster Komplikationen ausgesetzt.

Diese Defizite könnten durch die Verfügbarkeit funktioneller SA-Knotenzellen (synonym: Sinusknotenzellen, Knotenzellen, Herz-Schrittmacherzellen) für Transplantationen oder durch ihre *de novo*-Erzeugung *in vivo* vermieden werden. Knotenzellen sind durch ihr niedriges Membranpotential, die diastolische Depolarisation und niedrige Aufstrichgeschwindigkeiten charakterisiert. Mehrere unterschiedliche lonenströme sind an der diastolischen Depolarisation und den Aktionspotentialen im SA-Knoten beteiligt, unter anderem der Schrittmacherstrom I_{f}. Dieser Strom wird durch die durch zyklische Nukleotide regulierten HCN-Kanäle getragen. Die cAMP-Bindungsstelle am HCN-Kanal ermöglicht die Modulation der Aktivierung durch Katecholamine und diese Eigenschaft könnte die autonome Regulierung des Schrittmachermechanismus steuern [1]. Die Isoformen des Kanals, als HCN1 bis HCN4 bezeichnet, werden durch vier Gene kodiert. Die bei weitem vorherrschende Isoform im SA-Knoten ist HCN4 [2][3].

Um biologische Schrittmacherzellen für zukünftige Therapien zu erhalten, wurden zwei Ansätze verfolgt:
- einerseits ist es das Ziel, schlagende Herzmuskelzellen über eine genetische Manipulation *in situ* in Herz-Schrittmacherzellen umzuwandeln ("direkte Reprogrammierung"). In diesem Zusammenhang führte TBX3, ein elementarer früher Transkriptionsfaktor, zu Zellen mit unvollständigen Schrittmachereigenschaften[4]. Kürzlich wurde berichtet, dass eine virale Überexpression von Tbx18, einem Mitglied derselben Transkriptionsfaktorfamilie, die Reprogrammierung von Kammermyokard zu Sinusknotenzellen ermöglichte [5]. Allerdings benötigt diese Vorgehensweise virale Vektoren, was eine räumliche und zeitlich regulierbare Expression des Reprogrammierungsfaktors zur Simulation der *in vivo* Situation des sich entwickelnden Embryos verhindert. Darüber hinaus war der Wirkungsgrad sehr niedrig. [5]. Des Weiteren ist gezeigt worden, dass Tbx18 nur transient im Kopfteil des sich entwickelnden Sinusknotens exprimiert wird, während der im Gegensatz zur direkten Reprogrammierung hier (d.h. im erfindungsgemäßen Ansatz) für die gezielte Differenzierung eingesetzte Faktor Tbx3 permanent im gesamten Sinusknoten *in vivo* exprimiert wird [6].
- Ein weiterer Ansatz stützt sich auf die Transplantation von *in vitro* erzeugten "biologischen Schrittmachern", die aus pluripotenten Stammzellen (Stammzelle= S-Zelle oder SC "stemcell") wie embryonalen Stammzellen (ESCs oder ES-Zellen) oder induzierten pluripotenten Stammzellen (iPSCs) gewonnen wurden [7][8]. In diesem Zusammenhang ist postuliert worden, dass die niedermolekulare Verbindung EBIO die Bildung von Knotenzellen aus murinen ES-Zellen in einem gewissen Umfang steigert[9]. Allerdings wurde in der Publikation die eigentliche Fähigkeit der Zellen, ventrikuläre Herzmuskelzellen zu stimulieren, nicht angesprochen und ebenso waren die Schlagfrequenzen der Zellen (zu) niedrig. Außerdem wurde auf der elektrophysiologischen Ebene nicht zwischen relativ ausgereiften Schrittmacherzellen und dem ebenfalls spontan kontrahierenden frühen/intermediären Zelltyp unterschieden [9][10][11].

Kürzlich ist berichtet worden, dass Sinusknotenzellen aus einer über den Nachweis der Alcam-Expression (CD166) gereinigten Zellpopulation entstehen können. Hier ist jedoch die Übertragbarkeit auf das humane System nicht klar, da die Spezifität von Oberflächenmarkern zwischen den Spezies häufig nicht konserviert ist [12].

Kensah et al. beschreiben die genetische Modifikation muriner induzierter pluripotenter Stammzellen (iPSCs) durch Einbringung eines Zeocin-Resistenz Genes unter Kontrolle eines alpha-MHC(MYH6)-Promoters [17]. In der WO 2013/070952 A1 werden zwei Ansätze offenbart: Die Zellprogrammierung embryonaler Stammzellen (ES-Zellen), die ausschließlich mit Shox2 umgesetzt wurde, und das Umprogrammieren von ventrikulären Kardiomyozyten mit TBX18. Bakker et al. [4] beschreiben die direkte Reprogrammierung von kardialen Myozyten in schrittmacher-artige Zellen unter Verwendung von TBX3, d.h. eine unmittelbare Umwandlung von ausdifferenzierten in (andere) ausdifferenzierte Zellen. Otsuji et al. [18] beschreiben eine verbesserte Ausreifung in kontrahierenden Kardiomyozyten, welche aus humanen embryonalen Stammzellen erhalten wurden.

Aufgabe der vorliegenden Erfindung war es, Herz-Schrittmacherzellen (Sinusknotenzellen) mit verbesserten Schrittmachereigenschaften bereitzustellen, die in vitro aus Stammzellen gewonnen werden und dabei in einer hohen Ausbeute erhalten werden.

Diese Aufgabe wurde mit einem Verfahren gemäß Anspruch 1 gelöst. Weitere bevorzugte Ausführungsformen sind in den abhängigen Ansprüchen offenbart. In anderen Worten wurde diese Aufgabe durch ein Verfahren zur Erzeugung von Sinusknotenzellen ("Herz-Schrittmacherzellen") aus Stammzellen gelöst, bei welchem eine Nukleinsäure in Stammzellen eingebracht wird, wodurch diese einen TBX3-Transkriptionsfaktor exprimieren, oder ein TBX3-Protein in die Stammzellen eingebracht wird, wobei das Verfahren dadurch gekennzeichnet ist, dass zusätzlich ein Konstrukt zur Expression eines Antibiotikum-Resistenz-Gens, welches durch einen alpha-MHC (MYH6)-Promoter gesteuert wird, eingebracht wird und die resultierenden Stammzellen in Gegenwart des Antibiotikums differenziert werden. "Resultierende Stammzellen" meint Stammzellen, die sowohl TBX3 (Nukleinsäure oder Protein) als auch das Konstrukt aufweisen.

Dies stellt eine weiterentwickelte Art von Alternative der von den Erfindern kürzlich eingeführten gezielten Differenzierung von ES-Zellen zu spezifischen Kardiomyozyten-Subtypen über einzelne Transkriptionsfaktoren dar[13][14][11]. "Art von Alternative" deshalb, weil bisher andere Subtypen im Fokus standen.

Durch die Übertragung der Vorgehensweise, d.h. der gezielten Differenzierung über TBX3, konnte gezeigt werden, dass dies bereits zu einer Verdopplung funktioneller Schrittmacherzellen führt. Dies reicht allerdings immer noch nicht aus, um reine Populationen dieser Zellen zu erhalten. Dies korrelliert mit dem Unvermögen von TBX3 zur funktionellen Reprogrammierung als einzelner Faktor [4].

Erfindungsgemäß wurde daher die Methode mit einer Antibiotikaselektion auf der Basis des Myh6-Promotors[15] kombiniert. Dadurch wurden Zellaggregate erhalten, die ausschließlich aus spontan schlagenden Kardiomyozten-Zellen (KM-Zellen) bestanden und die eine Schlagfrequenz aufwiesen, die zum ersten Mal in der Nähe von der eines (Mäuse)herzens liegt.

Von diesen Zellen zeigten über 60%, insbesondere mehr als 70%, höchst bevorzugt über 80 % den gewünschten Typ der Schrittmacherzellen (bezogen auf die erzeugten Kardiomyozyten-Subtypen) mit einer vollständigen Funktionalität auf den Ebenen der Proteinexpression, der elektrophysiologischen Parameter und der Ca²⁺-Transienten sowie deren Potenz zur stabilen *ex vivo* Stimulation, insbesondere muriner, Herzmuskelkulturen. In der Literatur wird teilweise bereits eine 60%-ige Ausbeute erwähnt, dabei bleibt allerdings unerwähnt, dass nicht unterschieden wurde zwischenfrühen/intermediären Zellen und differenzierten Schrittmacherzellen. Erstere schlagen auch spontan -wie Herz-Schrittmacherzellen- haben aber ansonsten abweichende Eigenschaften, die sie als Herz-Schrittmacherzellen ungeeignet machen. (d.h. sie stehen am Anfang der Differenzierung und können/werden noch alle Subtypen, also neben Schrittmacherzellen auch atriale und ventrikuläre Zellen hervorbringen.)

Die erfindungsgemäß erhaltenen Zellen bzw. Zellaggregate, die auch als "induzierte sinuatriale Zellkörper(chen)" (iSABs) bezeichnet werden, wurden darüber hinaus unter Verwendung von RNA-Seq bezüglich ihres gesamten mRNS-Expressionsmusters analysiert, was die Resultate bestätigte. Somit resultiert die Erfindung in hoch angereicherten Populationen von Schrittmacherzellen, die aus Stammzellen abgeleitet wurden, welche alle Eigenschaften aufweisen, die für diesen Zelltyp charakteristisch sind, was eine große Bedeutung für eine zukünftige Zelltherapie und eine *in vitro*-Evaluation von Arzneimitteln haben wird.

Wenn eine Nukleinsäure zur Expression eines TBX3-Transkriptionsfaktors in die Stammzellen eingebracht wird, wird diese vorzugsweise ausgewählt aus TBX3-DNS, insbesondere TBX3-cDNS; oder TBX3-RNS, insbesondere TBX3-mRNS. Im Rahmen der RNS können TBX3-mRNS in die Stammzellen transfiziert werden, wobei dies keine stabile Gen-Modifikation ergibt. Alternativ können mikro-RNS eingebracht werden, die endogenes TBX zur Expression bringen. In einer bevorzugten Ausführungsform der Nukleinsäure-Einbringung wird TBX3-DNS, insbesondere TBX3-cDNS mittels Vektor, insbesondere mittels (Über-)Expressionsvektor, eingebracht. Für das TBX3 ist TBX3-cDNS bevorzugt. D.h. eine bevorzugte Variante ist die Einbringung von TBX3-cDNS mit Überexpressionsvektor. Das TBX3-Protein verursacht ebenfalls keine (stabile) Gen-Modifikation.

Eingesetzt werden humane oder nicht-humane Nukleinsäuren oder Proteine, wobei diejenigen humaner Herkunft bevorzugt sind.

Hinsichtlich der eingesetzten Stammzellen werden multipotente oder pluripotente, vorzugsweise pluripotente, Stammzellen eingesetzt. Die Stammzellen könnten ausgewählt werden aus humanen oder nicht-humanen embryonalen Stammzellen oder humanen oder nicht-humanen induzierten Stammzellen oder humanen induzierten Stammzellen oder parthenogenetischen Stammzellen oder spermatogonialen Stammzellen auszuwählen. Im Rahmen der vorliegenden Erfindung handelt es sich um nicht-humane embryonale Stammzellen oder nicht-humane induzierte Stammzellen oder humane induzierte Stammzellen oder parthenogenetische Stammzellen oder spermatogoniale Stammzellen, besonders bevorzugt nicht-humane embryonale Stammzellen oder nicht-humane induzierte Stammzellen oder humane induzierte Stammzellen. Humane embryonale Stammzellen sind explizit ausgenommen.

Die erfindungsgemäße Antibiotikaselektion auf der Basis des Myh6-Promotors nutzt vorzugsweise ein Antibiotikum-Resistenz-Gen ausgewählt aus Aminoglykosid-Antibiotikum-Resistenz-Gen, mehr bevorzugt aus Neomycin- und Puromycin-Resistenz-Gen, höchst bevorzugt Neomycin-Resistenz-Gen. Das für die Selektion eingesetzte Antibiotikum ist entsprechend ausgewählt aus Aminoglykosid-Antibiotikum, insbesondere aus Neomycin und Puromycin. "Entsprechend ausgewählt" bedeutet, dass immer das zum Resistenz-Gen passende Antibiotikum eingesetzt wird, beispielsweise beim Neomycin-Resistenz-Gen wird anschließend mit Neomycin selektiert.

Erzeugt werden in jedem Fall Herz-Schrittmacherzellen (human oder nicht human), wobei humane Herz-Schrittmacherzellen bevorzugt sind. Hierfür werden humane Stammzellen mit vorzugsweise humanem Protein oder humaner Nukleinsäure kombiniert. Kreuzkombinationen, wie beispielsweise die Einführung humaner Proteine oder humaner Nukleinsäure in nicht-humane, z.B. murine, Stammzellen ist ebenfalls möglich, auch die reine Kombination der nicht-humanen Vertreter zur Erzeugung nicht humaner Herz-Schrittmacherzellen.

Wie nachfolgend noch im Detail erläutert, zeigen die erfindungsgemäßen, *in vitro* aus Stammzellen erzeugten Sinusknotenzellen (synonym verkürzt auch: erfindungsgemäße Sinusknotenzellen bzw. erfindungsgemäß aus Stammzellen erzeugten Sinusknotenzellen) bei in-Kontakt-bringen mit Pharmazeutika das erwartete Verhalten: beispielsweise verursachen sowohl der HCN-Kanal-Blocker ZD-7288 als auch der Muskarinrezeptoragonist Carbachol eine signifikant verringerte Schlagfrequenz. Die Verabreichung des β-Adrenorezeptoragonisten Isoprotenerol hingegen führt zu einer erhöhten Schlagfrequenz. Da die erfindungsgemäßen, aus Stammzellen erzeugten Sinusknotenzellen auf die verbreichten Pharmazeutika genauso wie Sinusknotenzellen *in vivo* reagierten, sind sie als Modell für normale Sinusknotenzellen geeignet. *In vitro* aus Stammzellen erzeugte Sinusknotenzellen, insbesondere die erfindungsgemäß (*in vitro*) aus Stammzellen erzeugten Sinusknotenzellen werden daher u.a. auch zur *in vitro*-Evaluation von Arzneimitteln eingesetzt. Hierunter ist synonym die Untersuchung von Wirkstoffen, d.h. potentiellen Arzneimitteln, auf ihre tatsächliche Eignung als solche zu verstehen ("*in vitro* drug testing"). Potentielle Kandidaten für neue Arzneimittel müssen in vorgeschriebenen präklinischen und klinischen Studien auf ihre Qualität, Unbedenklichkeit und Wirksamkeit getestet werden, bevor sie von den Arzneimittelbehörden für die Vermarktung zugelassen werden. Da bisher die biochemischen als auch die cheminformatischen Voruntersuchungen keine finale Gewissheit liefern, wie sich ein neuer Wirkstoff *in vivo* verhält, müssen neue Wirkstoffe in präklinischen Studien geprüft werden, was Tierversuche in großem Aufwand bisher unabdingbar machte. Der Einsatz von *in vitro* aus Stammzellen erzeugten Sinusknotenzellen, insbesondere der erfindungsgemäßen Sinusknotenzellen kann hierbei zumindest helfen, das Maß an Tierversuchen zu reduzieren. Ebenso werden *in vitro* aus Stammzellen erzeugte Sinusknotenzellen, insbesondere die erfindungsgemäßen Sinusknotenzellen im Vorfeld der Präklinik eingesetzt, da auch bei der Suche nach Wirkstoffkandidaten und bei der Toxizitätsuntersuchung oftmals zellbasierte *in vitro* Assays genutzt werden können, aufgrund der Tatsache, dass diese essentiale Aspekte der *in vivo* Pharmakologie und Toxikologie wiederspiegeln können.

Ebenso nutzbringend werden *in vitro* aus Stammzellen erzeugte Sinusknotenzellen, insbesondere die erfindungsgemäß aus Stammzellen erzeugten Sinusknotenzellen zur Herzgewebekonstruktion und/oder Herzgewebezüchtung, insbesondere zu Implantationszwecken, aber auch für die *de novo* Erzeugung *in vivo* verwendet. Insbesondere im Fokus ist hier die Erzeugung zellbasierter biologischer Herz-Schrittmacher.

Die der Erfindung zugrunde liegenden Untersuchungen werden nachfolgend im Detail vorgestellt:

### Erzeugung von stabilen TBX3-überexprimierenden ES-Zelllinien

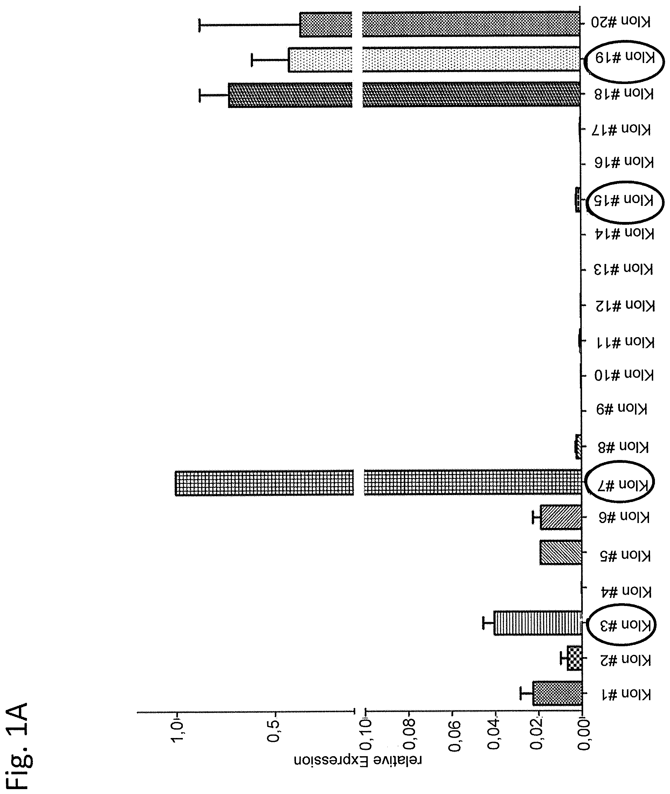

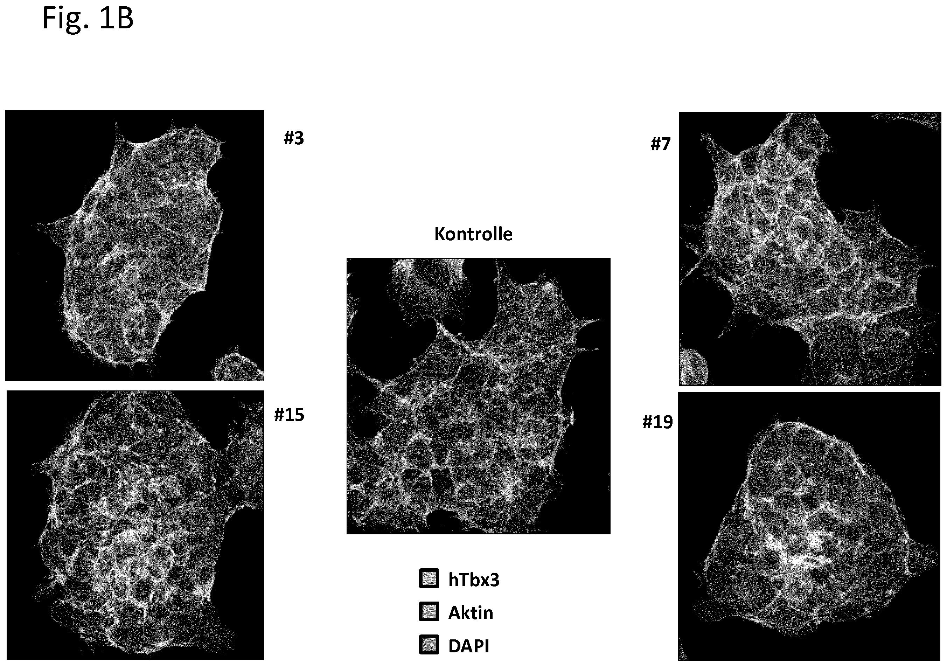

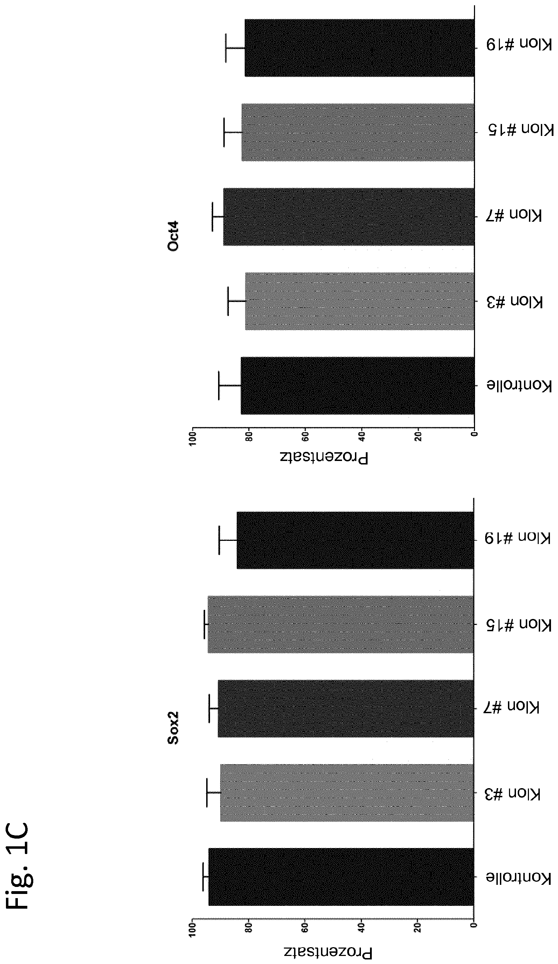

Auf der Basis der hohen Konservierung der TBX3-Proteine bei Wirbeltieren wurde das humane TBX3 in murinen ES-Zellen aufgrund seiner hier spezifischen Nachweisbarkeit eingesetzt. Für die Überexpression in ES-Zellen wurde die humane TBX3-cDNS in pEF-DEST51 (Invitrogen) eingesetzt. Aus 20 unabhängigen Klonen wurden vier Klone, die das gesamte Spektrum von einem niedrigen bis zu einem hohen Grad der TBX3-mRNS-Überexpression repräsentieren, anhand der Ergebnisse der qRT-PCR ausgewählt (Abb. 1A). Diese Überexpressionslevel wurden auf Proteinebene bestätigt (Abb. 1B). In FACS-Analysen wurde kein Hinweis auf eine Beeinflussung der TBX3-Überexpression auf die den prozentualen Anteilder Pluripotenz-Marker Oct-4/Pou5f1-positiven und Sox2-positiven Zellen gefunden (Fig. 1C). Dies entspricht einem normalen undifferenzierten Koloniewachstum in einem LIF enthaltenden Medium (Abb. 1B) und stimmt mit den vorher erhaltenen Ergebnissen für MESP1- und NKX2-5-Überexpression überein [13][14][11]. Daraus wurde geschlossen, dass analog zu diesen Faktoren, TBX3 alleine nicht die Potenz besitzt, um eine Differenzierung von ES-Zellen zu induzieren.

### Auswirkung der TBX3-Überexpression auf Ausbeute und Subtypen der aus ES-Zellen abgeleiteten spontan schlagenden Kardiomyozyten

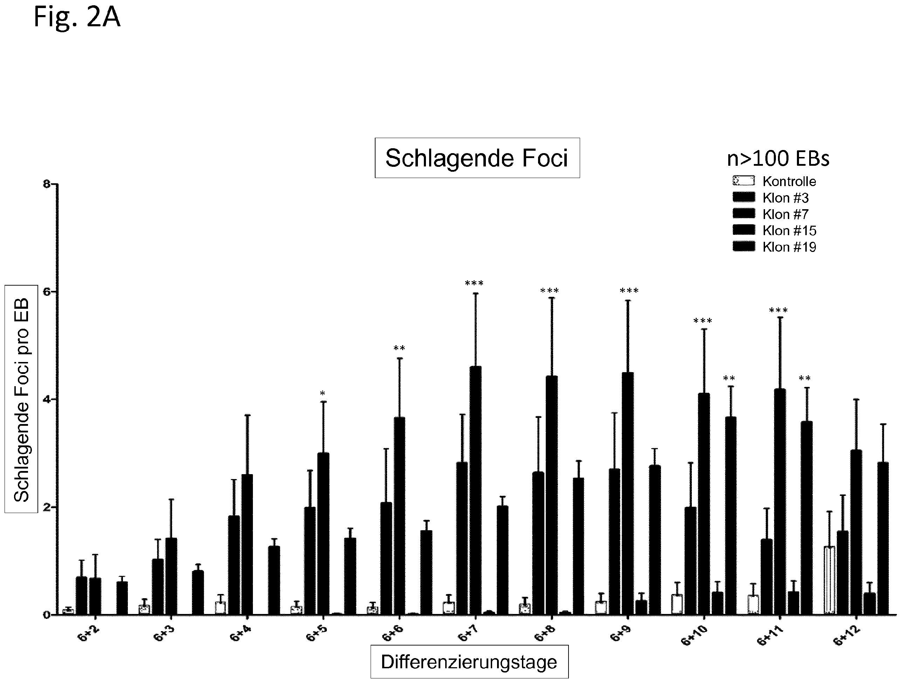

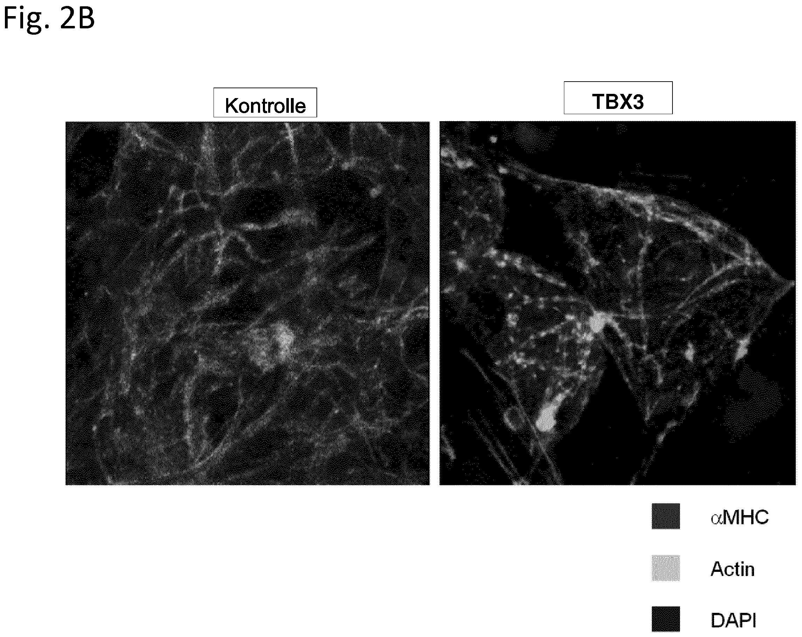

ES-Zellklone mit hoher und mittlerer TBX3-Überexpression begannen während der Differenzierung früher zu kontrahieren und zeigten ungefähr die 5- bis 10-fache Anzahl an kontrahierenden Arealen (Abb. 2A). Diese erhöhte kardiale Differenzierung ist ähnlich zu der Wirkung der MESP1- und NKX2-5-Überexpression, die kürzlich von den Erfindern beschrieben wurde [13][14][11]. Übereinstimmend mit ihrer gesteigerten Schlagaktivität zeigten TBX3-überexprimierende Kardiomyozyten normale Expressionsmuster des sarkomeren Markers Myh6 (Abb. 2B).

Für die weitere Validierung der Funktionalität und Bestimmung der Anteile der Subtypen der Kardiomyozyten wurden die elektrophysiologischen Eigenschaften mit der Einzelzell-Patch-Clamp-Technik und der Messung der Dichte der HCN- (oder Funny-)Kanäle an Tag 18 der Differenzierung wie beschrieben analysiert [13][14][16].

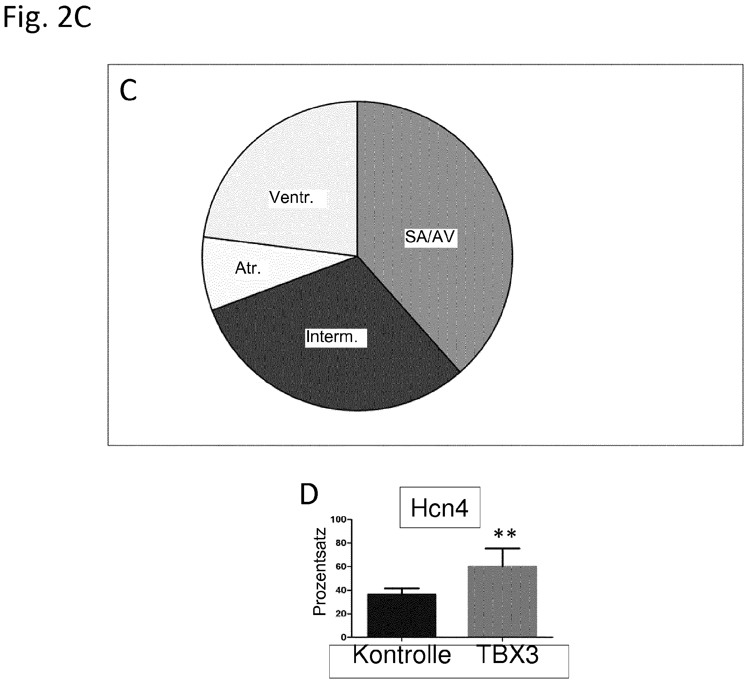

Generell sind alle beschriebenen Subtypen, die an isolierten schlagenden Kardiomyozyten während der EB-Entwicklung erhalten werden, in den Präparationen der TBX3-Zellklone vorhanden, nämlich ventrikelartige, vorhofartige und SA/AV- (schrittmacherartige) Zellen sowie frühe/intermediäre Zellen (Abb. 2 C; Zusatz-Tabelle 1). Die durch die unterschiedlichen Zelltypen generierten Aktionspotentiale unterschieden sich nicht signifikant zwischen den TBX3- und den Kontrollzellen bezüglich ihrer individuellen Parameter wie etwa MDP, DDR, Aufstrichgeschwindigkeit und der Dauer der AP-Plateauphase oder in Bezug auf ihre Reaktion auf β-adrenerge (Isoproterenol) oder muskarinerge (Carbachol) Stimulation, wodurch die Annahme einer korrekten Entwicklung von Kardiomyozyten gestützt wird (Abb. 2C; Zusatz-Tabelle 1).Es wurde jedoch ein ungewöhnlich hoher Anteil des schrittmacherartigen Subtyps bei den TBX3-überexprimierenden Zellen, die 38,5 % aller Kardiomyozyten stellten, gefunden. Diese Zellen besaßen keine Plateauphase, verfügten über eine hohe Dichte an Funny-Kanälen (I_{f}), ein positives MDP von > -60 mV und zeigten die höchsten DDR-Werte, die normalerweise 60 mV/s überstiegen. Im Gegensatz dazu besaßen sie die langsamste Aufstrich- und die schnellste Depolarisationsgeschwindigkeit (< 5 V/s) und den kleinsten positiven Overshoot mit einem Maximum von + 10 mV. Weiterhin reagierten die SA/AV-Zellen wie erwartet auf Isoproterenol, was zu einer Beschleunigung der AP-Rate führte. Übereinstimmend mit dem hohen Anteil an Schrittmacherzellen in den durch TBX3 gezielt differenzierten Kardiomyozyten war die Anzahl von Zellen, die den HCN4-Kanal exprimieren, welcher dem "Funny-Kanal" entspricht, erhöht (Abb. 2D).

Um die Ausbeute des SA/AV-Subtyps, der über die gezielte Differenzierung mittels TBX3 erhalten wurde, weiterzu erhöhen, wurde versucht, dieses Verfahren mit einer auf dem Myh6-Promotor basierenden Antibiotikaselektion zu kombinieren[15], da für letztere Methode kürzlich beschrieben wurde, dass sie besonders Schrittmacherzellen anreichert [17][18], allerdings mit einer sehr unbefriedigenden Ausbeute von nicht mehr als 40%.

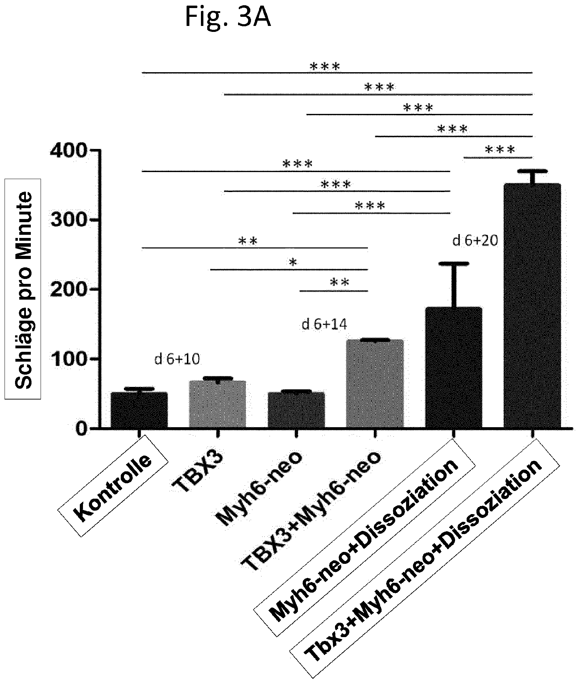

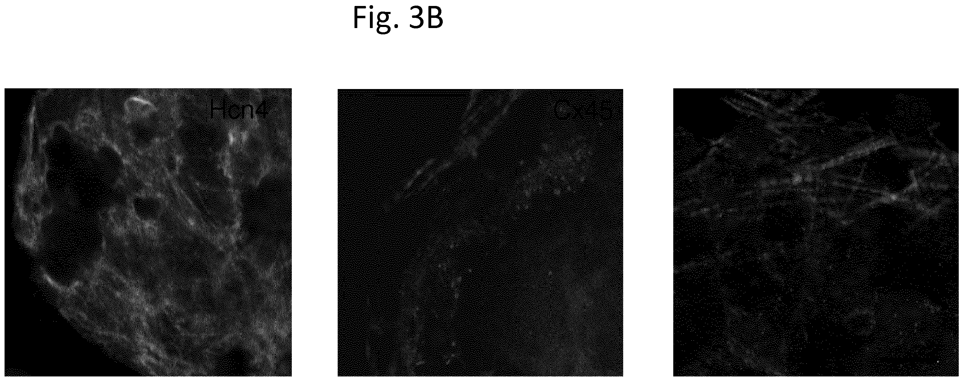

Dazu wurde zusätzlich das die Myh6-Neomycin-Kassette enthaltende Plasmid [15] in die TBX3-Klone eingeführt. Die Gabe des Antibiotikums (Neomycin) während der Differenzierung führte wie erwartet zu einer Anreicherung von schlagendem Gewebe in den durch Myh6-Promotor-gestützter Antibiotikaselektion (jedoch ohne Tbx3) erhaltenen Kontrollzellen, die als Antibiotika-selektierte kardiale Zellkörperchen (CardiacBodies) ("aCaBs") bezeichnet wurden. Auf die gleiche Weise wurden die doppelt transgenen Myh6-TBX3-Klone auf sich spontan kontrahierende Areale angereichert. Allerdings war bei den letztgenannten die Schlagrate der spontan schlagenden Zellen annähernd verdoppelt (Abb. 3A). Nach der Einführung eines zusätzlichen Dissoziationsschrittes wurden sowohl in den durch Antibiotika selektierten Myh6-Promotor-Kontrollzellen als auch in den doppelt transgenen Myh6-TBX3-Zellklonen eine weiter erhöhte Schlagrate erhalten. Bei den letztgenannten Zellen wurden zum ersten Mal Zellaggregate erhalten, die mit 300 - 400 bpm kontrahierten, was nahe an der Schlagfrequenz muriner Herzen (∼500 bpm) ist und auf die Bildung von "induzierten sinuatrialen Zellkörpern" (iSABs) hinweist (Abb. 3A). Die Zellen innerhalb der iSABs waren für HCN4 sowie für die Connexine Cx45 und Cx30.2 positiv (Abb. 3B), was für Schrittmacherzellen charakteristisch ist [19, 20].

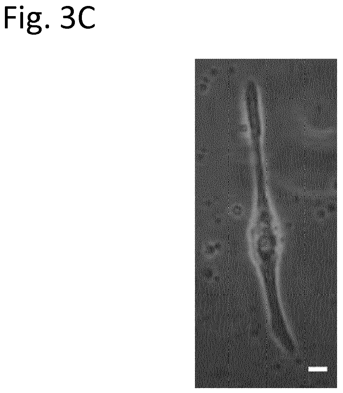

Die weitergehende Kultivierung der iSABs für drei Wochen auf mit Gelatine beschichteten Zellkulturschalen führte zu angewachsenen, hoch synchronisierten Zellschichten, die mit >350 bpm schlugen. Die Zellen wiesen die typische langgestreckte Form von Sinusknotenzellen auf (Abb. 3C).

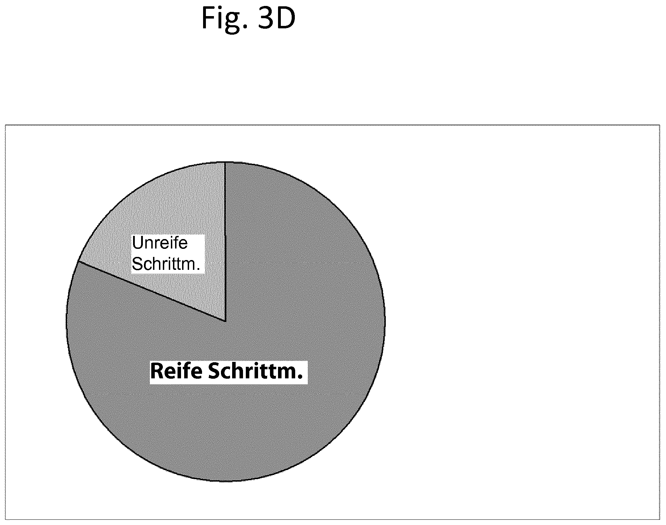

Zur Untersuchung der elektrophysiologischen Parameter der aus den iSABs abgeleiteten Kardiomyozyten wurde wiederum die Einzelzell-Patch-Clamp-Technik eingesetzt. Von den 65 analysierten Zellen entsprachen jetzt 53 Zellen (81,5 %) dem regelmäßig und schnell schlagenden Subtyp der Schrittmacherzellen und nur 12 Zellen (18,5 %) repräsentierten den irregulär und langsam schlagenden myokardialen Subtyp. Weiterhin zeigten 43 von den 53 als Subtyp der Schrittmacherzellen klassifizierten Zellen AP-Parameter reifer Schrittmacherzellen, während 10 von diesen regelmäßig spontan schlagenden Zellen noch unreif waren (Abb. 3D, Zusatz-Tabelle 2). Obwohl sie eine kurze Plateauphase und ein negativeres MDP aufweisen, was sie von reifen Schrittmacherzellen unterscheidet, zeigen sie jedoch bereits die typischen Merkmale von Schrittmacherzellen, wie die Erzeugung regelmäßiger spontaner Aktionspotentiale mit einer schnellen DDR und weisen den typischen Schrittmacherstrom I_{f} auf (siehe z. B. die Ableitungen in Abb. 3B).

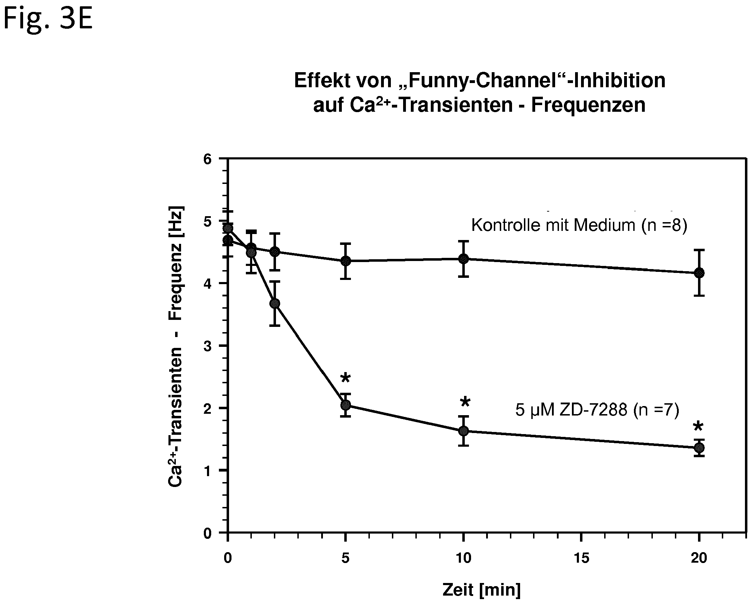

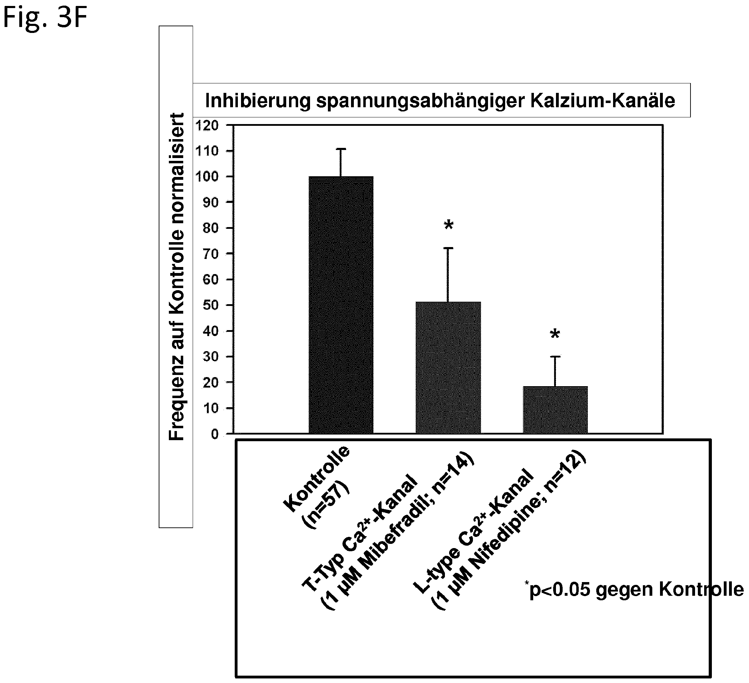

Zum weiteren Beweis ihrer Identität mit Schrittmacherzellen, wurde der Ca²⁺-Strom aus dem extrazellulären Raum und den intrazellulären Ca²⁺-Speichern charakterisiert, der durch die Ca²⁺-Kanäle im Sarkolemma oder durch die Freisetzung von Ca²⁺ aus den Speichern vermittelt wird. Die physiologische Funktionalität der im Sarkolemma sitzenden Kanäle bestimmen auch die Frequenz der Ca²⁺-Transienten. Als ein Merkmal von Schrittmacherzellen wurden die Zellen auf HCN-Kanäle überprüft, die die Frequenz der Ca²⁺-Transienten modulieren. Durch die Gabe des HCN-Kanal-Blockers ZD 7288 verringerte sich zeitabhängig die Frequenz der Ca²⁺-Transienten in den iSABs (Abb. 3E). Weiterhin basiert die Frequenz der Ca²⁺-Transienten ebenfalls auf der Aktivität der spannungsabhängigen T-Typ- und L-Typ-Ca²⁺-Kanäle. Entsprechend wurden die spontanen Frequenzen der Ca²⁺-Transienten in den aus iSAB abgeleiteten Zellen nach Inhibierung der L-Typ-Ca²⁺-Kanäle mit Nifedipin dramatisch verringert und die Inhibierung der T-Typ-Ca²⁺-Kanäle mit Mibefradil führte zu einer moderaten Verringerung (Abb. 3F).

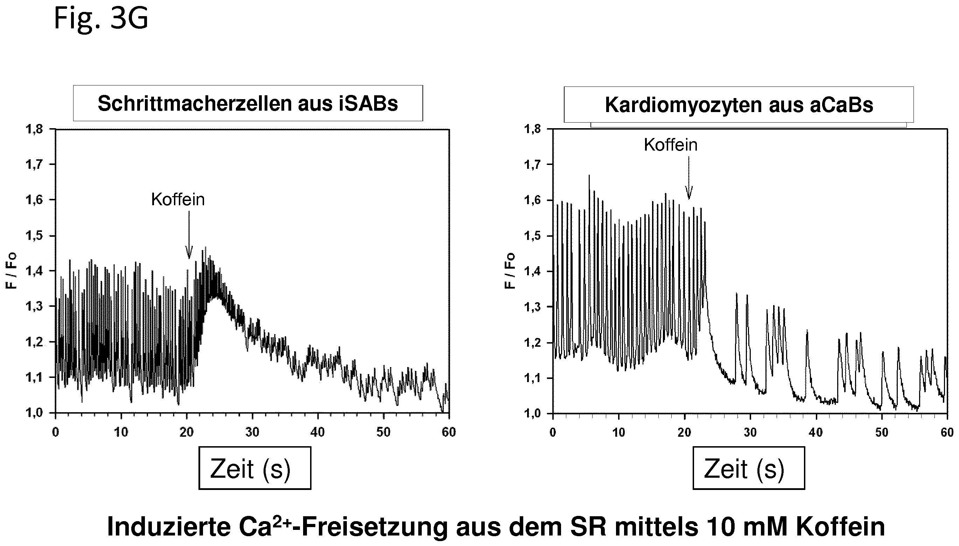

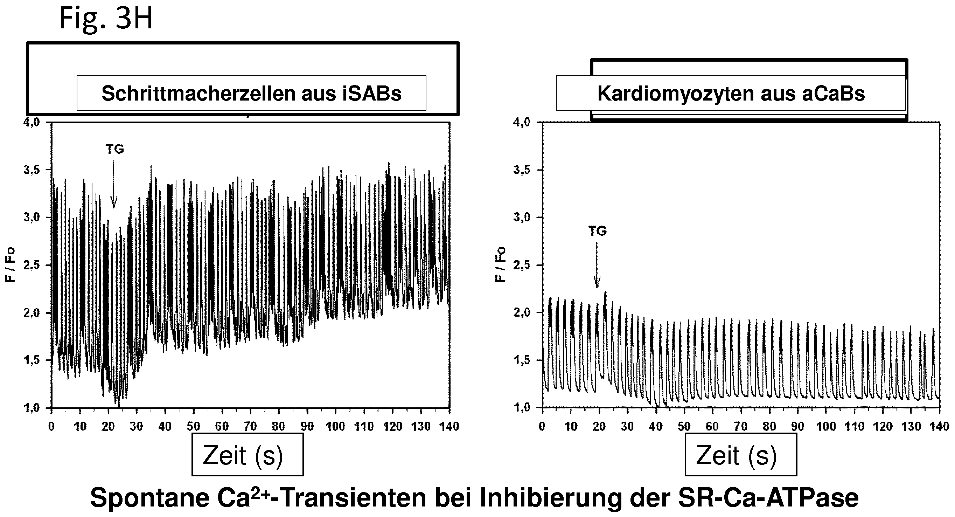

Ein funktionelles sarkoplasmatisches Retikulum (SR) kennzeichnet das Reifestadium der Kardiomyozyten. Somit spielt das Ca²⁺ aus dem SR eine bedeutende Rolle für die spontane Aktivität. Dieser Einfluss des aus dem SR stammenden Ca²⁺ auf spontane Ca²⁺-Transienten wird ersichtlich, wenn eine vollständige Freisetzung von Ca²⁺ aus dem SR mit Koffein oder eine Hemmung von SERCA mit Thapsigargin induziert wird. In den aus iSABs abgeleiteten Zellen erhöhte die Koffein-induzierte Ca²⁺-Freisetzung aus dem SR den diastolischen Ca²⁺-Spiegel vergleichbar zu einem Ca²⁺-Peak, jedoch mit erkennbaren spontanen Ca²⁺-Transienten im zeitlichen Verlauf des Ca²⁺-Peaks und mit unveränderten systolischen Ca²⁺-Werten (Abb. 3G). Eine ähnliche, durch Koffein induzierte Wirkung auf die SR-Ca²⁺-Freisetzung kann in den aus aCaBs abgeleiteten Zellen nicht nachgewiesen werden. Die Blockade der Wiederaufnahme von Ca²⁺ in das SR durch Thapsigargin resultierte nur in den aus iSABs abgeleiteten Zellen in einer Zunahme des diastolischen Ca²⁺-Spiegels (Abb. 3H).

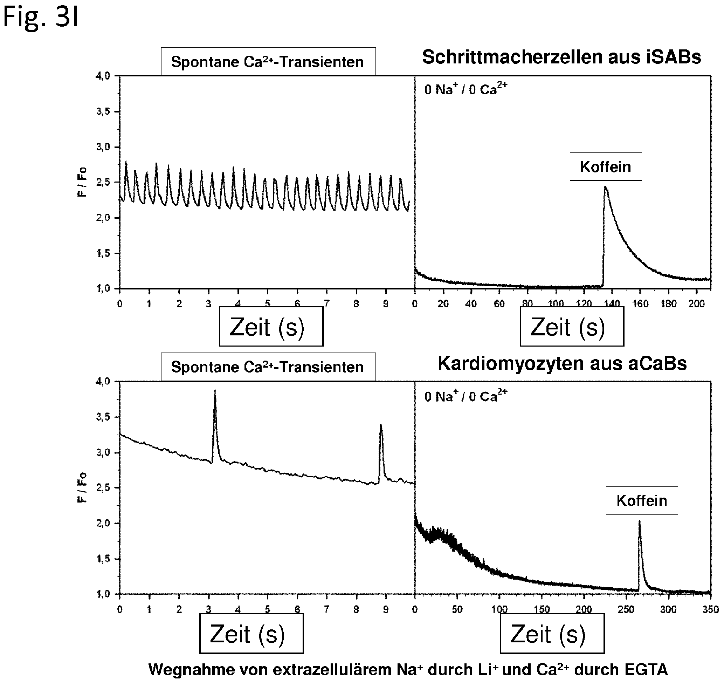

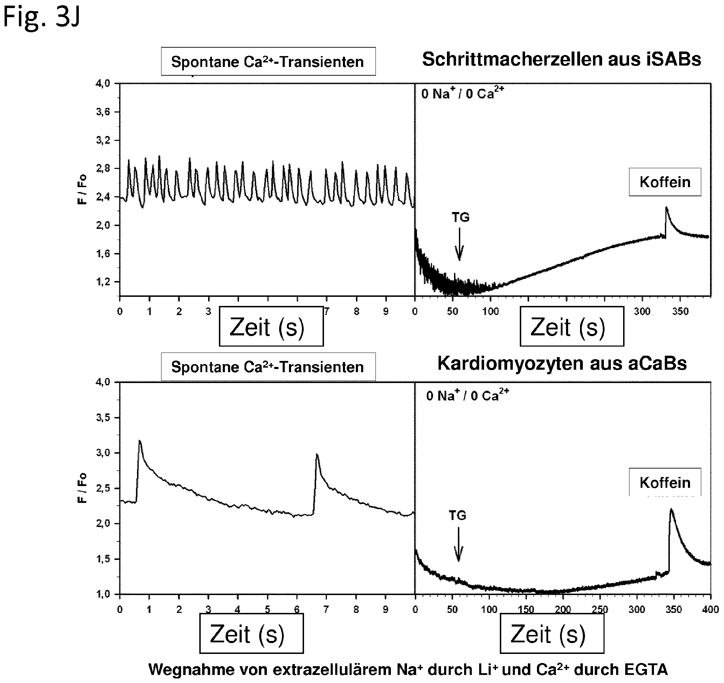

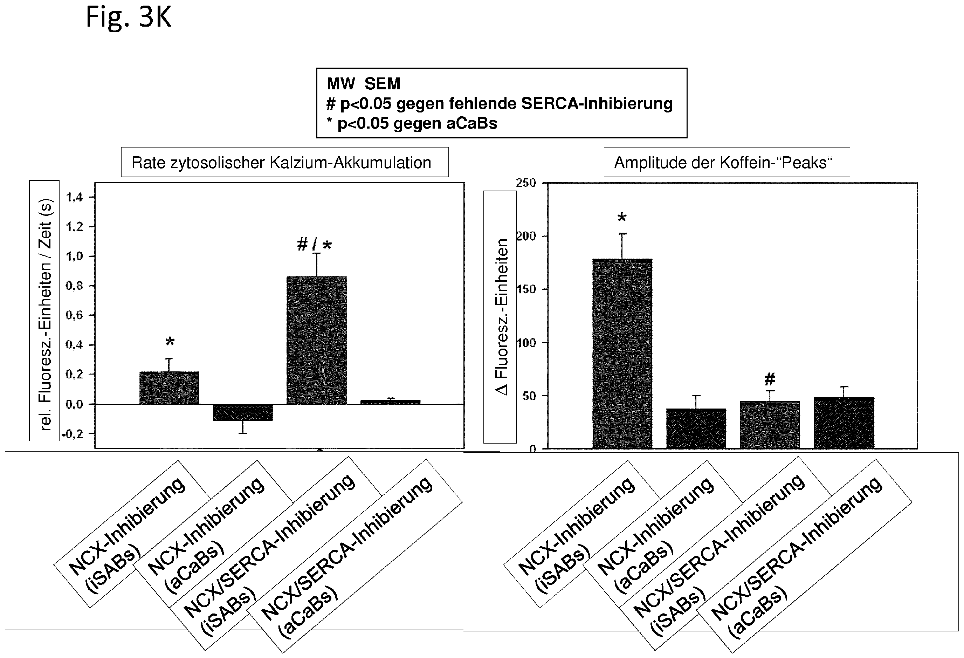

Der überwiegende Anteil des den Ca²⁺-Transienten zugrundeliegenden Ca²⁺stammt aus dem Extrazellulärraum. Deshalb führt ein Austausch des extrazellulären Ca²⁺ zu einer Aufhebung der Ca²⁺-Transienten. Wenn der sarkolemmale Ca²⁺-Einstrom durch die Blockierung des Na⁺/Ca²⁺-Austauschers und der Ca²⁺-Kanäle eliminiert wird, kann nur ein intrazellulärer Ca²⁺-Kreislauf detektiert werden. In diesem Fall induzierte die Zugabe von Koffein in den aus iSAB abgeleiteten Zellen einen Ca²⁺-Peak, der gegenüber dem in aus aCaBs abgeleiteten Zellen vierfach größer ist (Abb. 3I, K). Die zusätzliche Inhibierung der SERCA offenbarte unter diesen Bedingungen ein Leck im SR der aus iSABs abgeleiteten Zellen. Die Rate der intrazellulären Ca²⁺-Akkumulation ist im Vergleich zur Messung ohne SERCA-Inhibierung vierfach erhöht. Durch die Zugabe von Koffein 5 min nach der Inkubation mit Thapsigargin wurde der Ca²⁺-Peak um den Faktor 4 erniedrigt. Im Gegensatz dazu bleibt in den Kontrollzellen der Ca²⁺-Peak mit und ohne SERCA-Inhibierung identisch (Abb. 3J, K), wobei auch keine intrazelluläre Ca²⁺-Akkumulation festgestellt werden konnte.

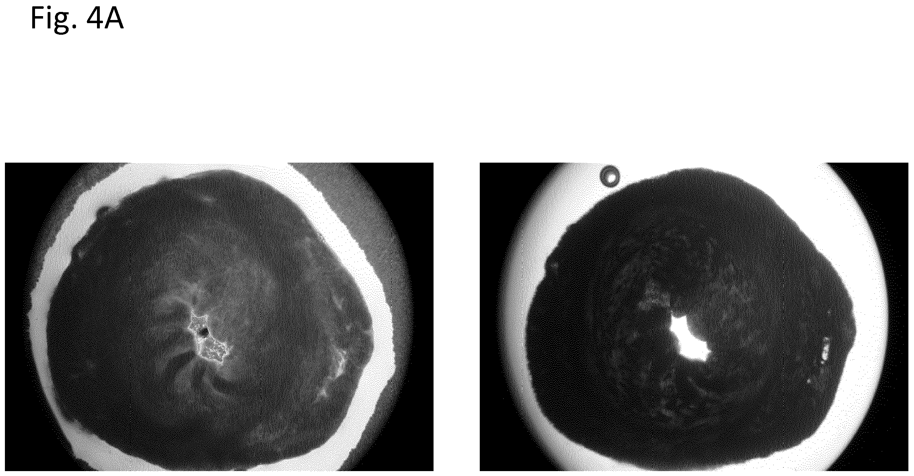

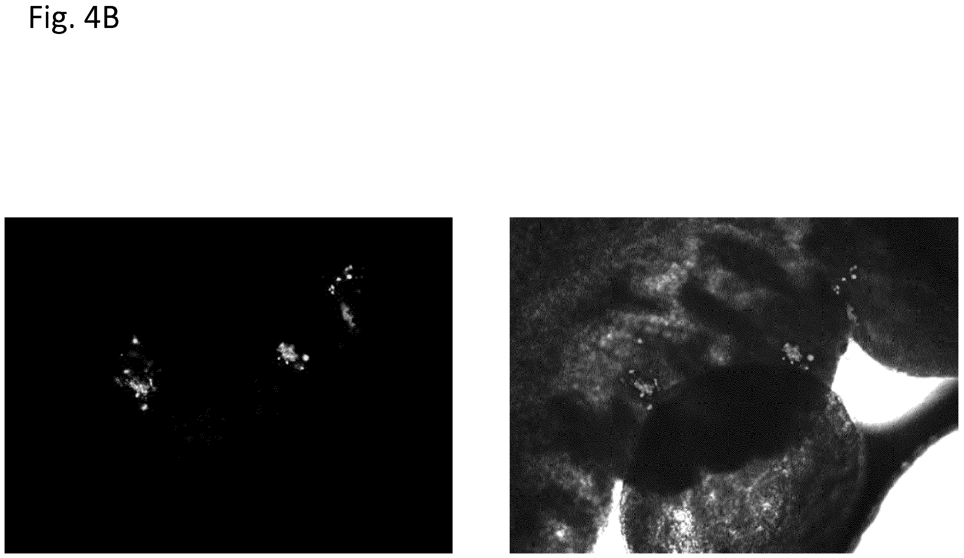

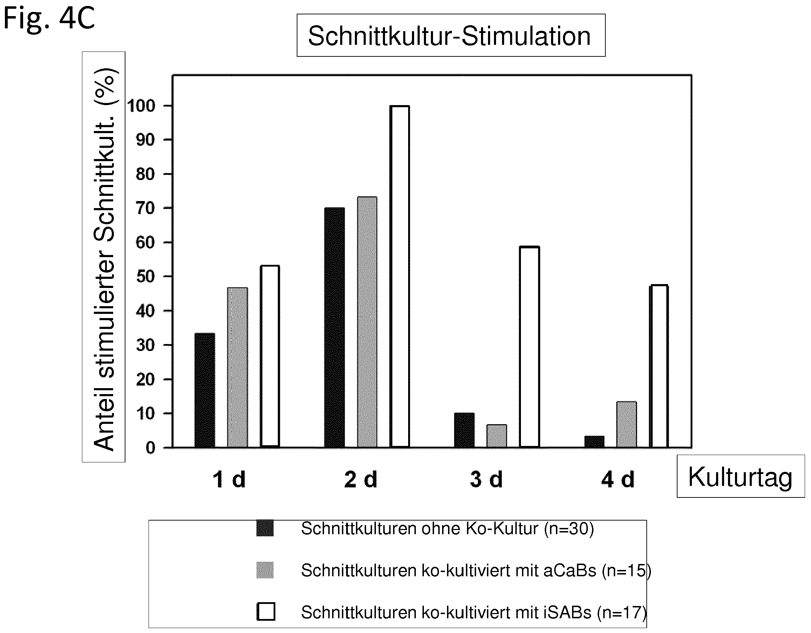

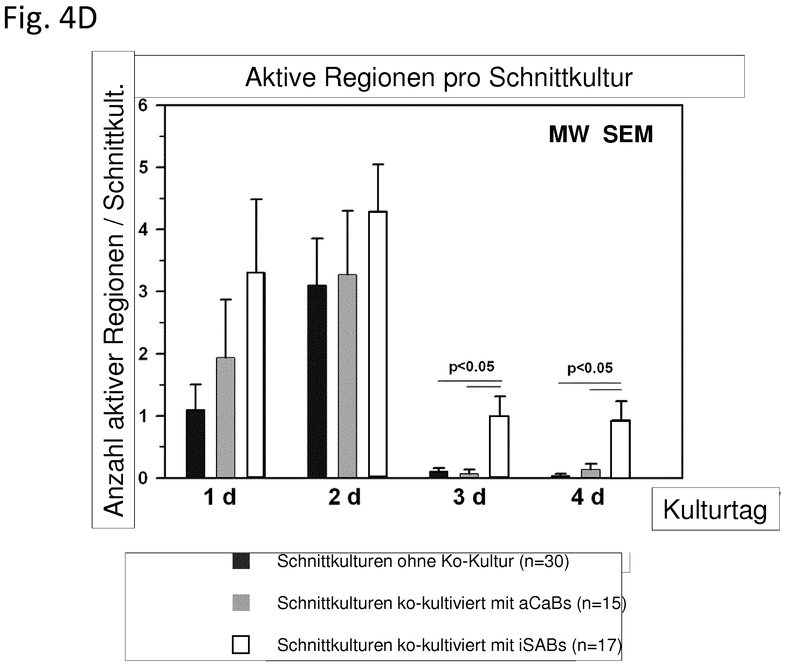

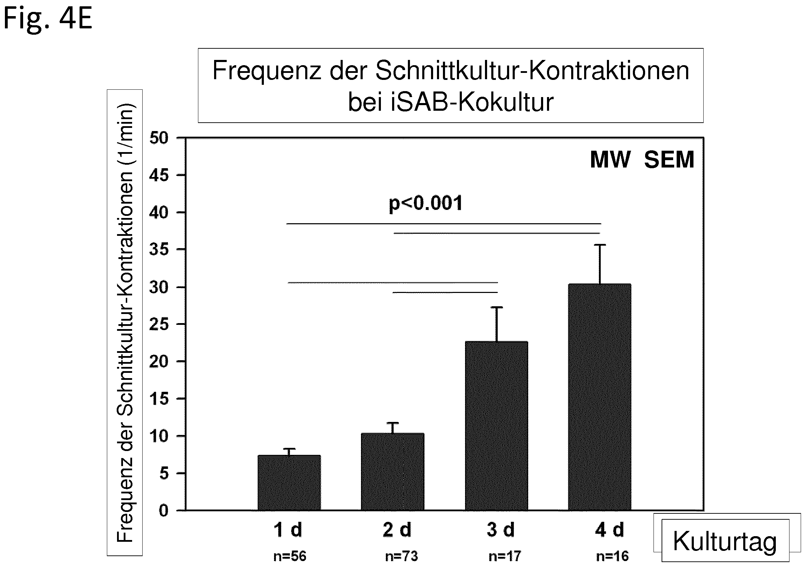

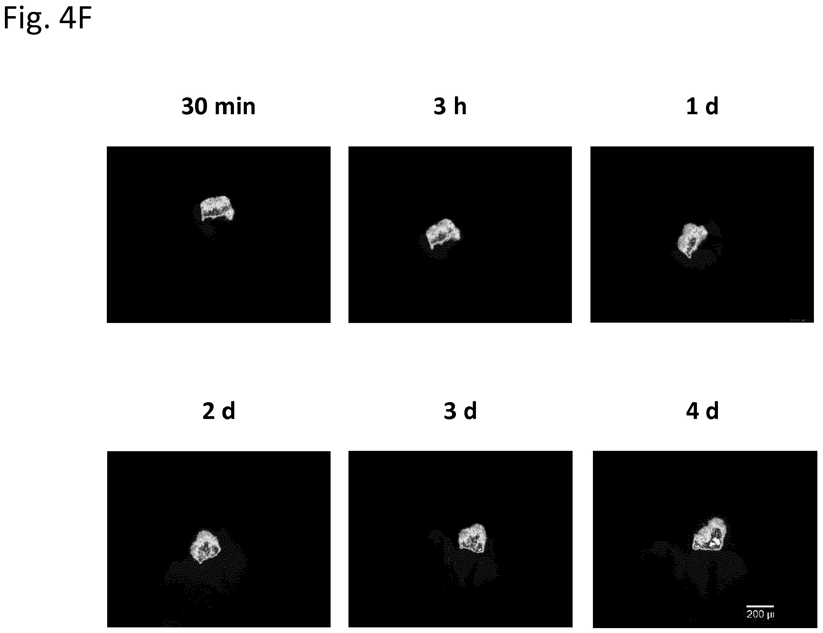

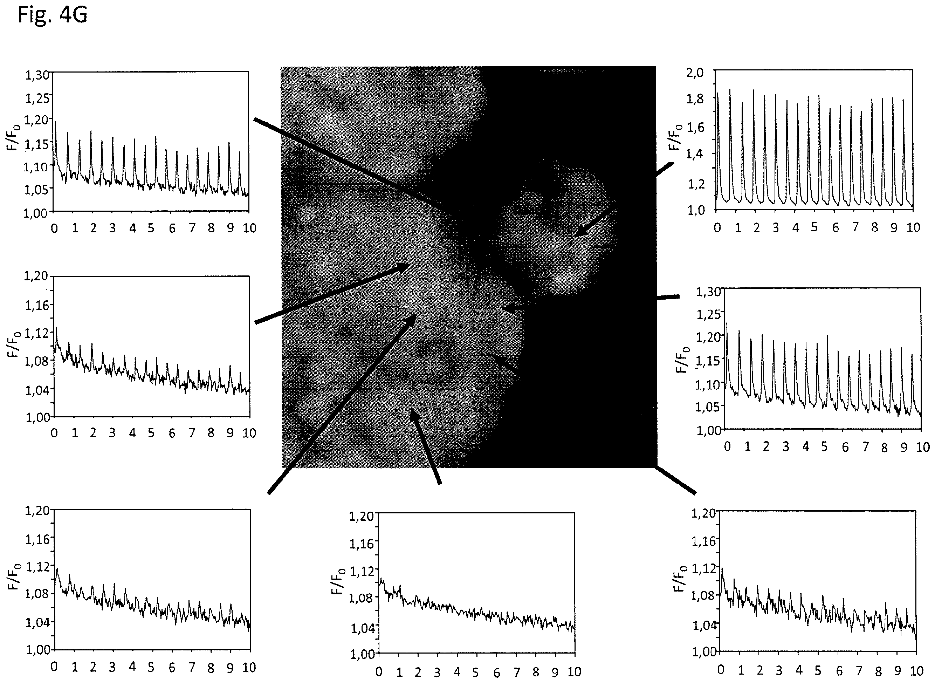

Um die iSABs letztendlich auf eine funktionelle Schrittmacheraktivität hin zu untersuchen, wurden die Vorteile eines *ex vivo*-Modells genutzt, das auf kultivierten Ventrikelschnitten von murinen Herzen basiert [21] (Abb. 4A). Während diese normalerweise unmittelbar nach der Präparation eine spontane Schlagaktivität aufweisen, kann bei ihnen über Elektroden eine stabile Kontraktion bis zu -60 bpm induziert werden und sie stellen dadurch ein ideales Testsystem für die Funktionalität der erzeugten iSABs dar. Wie aus der Markierung mit Dil ersichtlich, waren die auf den Schnitten ausgesäten iSABs in der Lage, sich an die Schnitte anzuheften und darauf fortzubestehen (Abb. 4B). Um die Wirkung der Aussaat der iSABs zu bestimmen, wurde zuerst die spontane Schnittaktivität in naiven Schnitten sorgfältig quantifiziert. Nachdem an Tag 2 nach der Schnitt-Präparation ein Maximum von ∼70 % der Schnitte mindestens ein Areal mit spontaner Schlagaktivität enthielt, nahm dieser Prozentsatz an den Tagen 3 und 4 dramatisch ab (Abb. 4C). Während die mit iSABs besäten Schnitte, im Gegensatz zu den mit aCaBs, bereits an Tag 1 und 2 eine ∼1,5-fach erhöhte Schlagaktivität zeigten, blieb ausschließlich auch nur deren Aktivität danach erhalten (Abb. 4C). Darüber hinaus enthielten diese Schnitte an Tag 3 und Tag 4 bedeutend mehr aktive Bereiche pro Schnitt im Vergleich zu den nicht besäten und den mit aCaB besäten Schnitten (Abb. 4 D). Weiterhin nahmen die Schlagfrequenzen der mit iSAB besäten Schnitte kontinuierlich und signifikant mehr als 4-fach von Tag 1 bis Tag 4 zu (Abb. 4E), dies wurde bei den unbesäten und den mit aCaB besäten Schnitten nicht beobachtet (dns). Die Beladung der iSABs mit Calcein vor der Überführung auf die Schnitte bestätigte die Bildung von Synzytien zwischen den iSAB und den Ventrikelzellen, wie es aus der Übertragung des Farbstoffs über die Zeit ersichtlich ist (Abb. 4F). Darüber hinaus ist die funktionelle Kopplung aus den synchronisierten Ca²⁺-Transienten zwischen iSABs und den Myokardzellen des Schnittes ersichtlich. Dadurch sind die Peaks der Ca²⁺-Transienten innerhalb des Schnittes kleiner, aber hoch synchron mit der spontanen iSAB-Aktivität und können innerhalb eines Radius von ∼200 µm festgestellt werden (Abb. 4G).

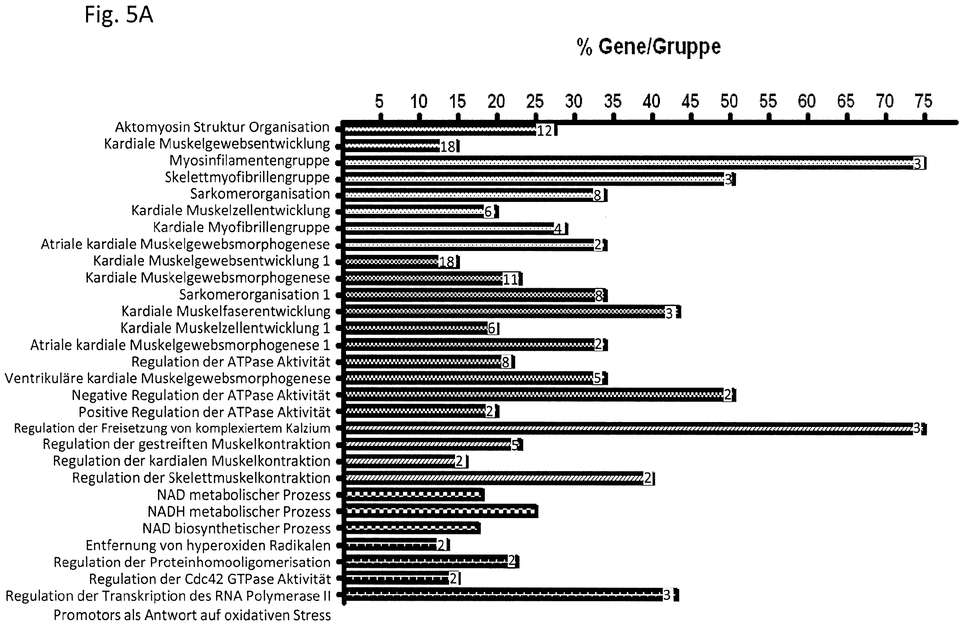

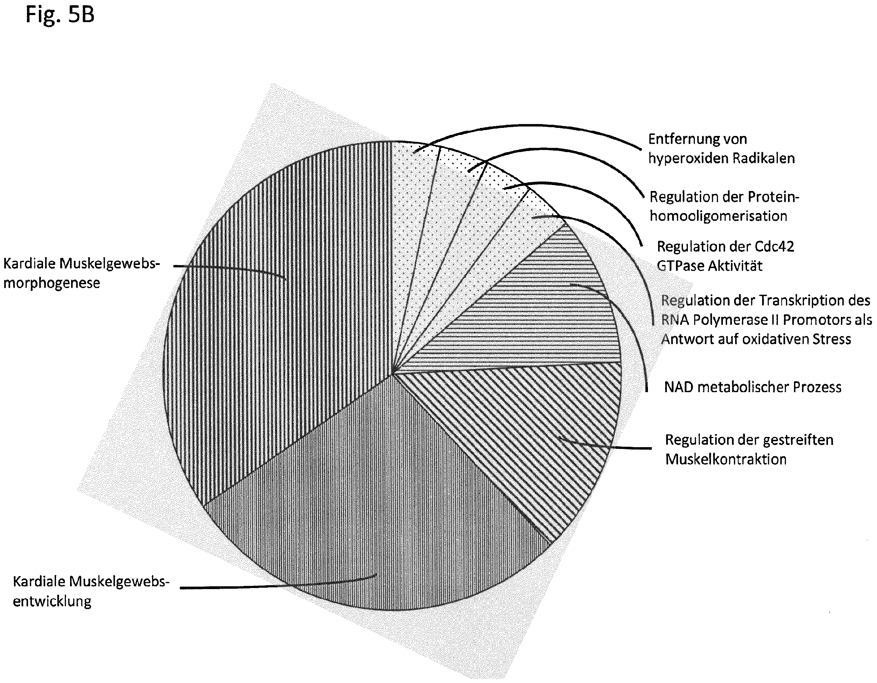

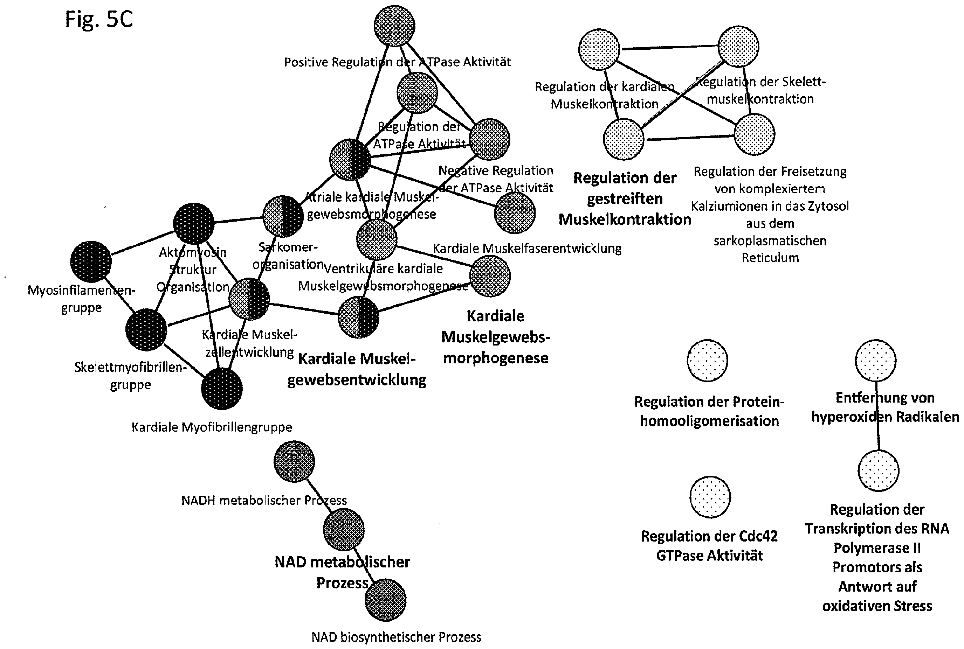

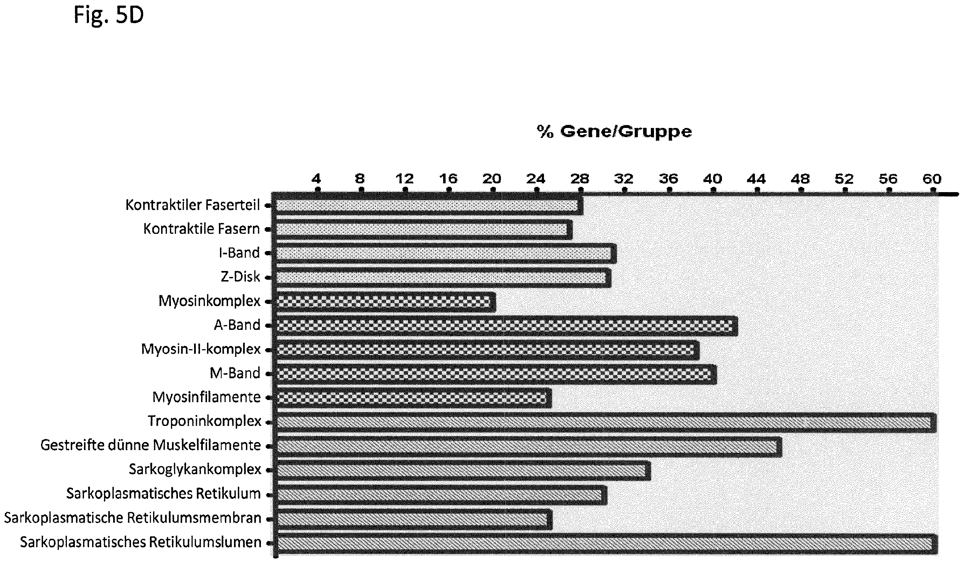

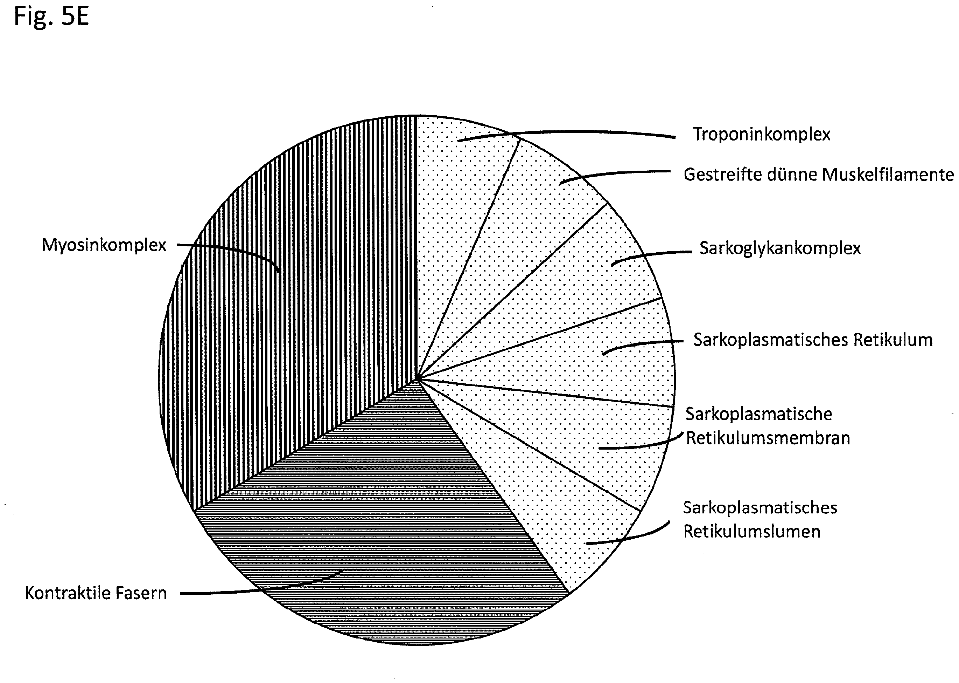

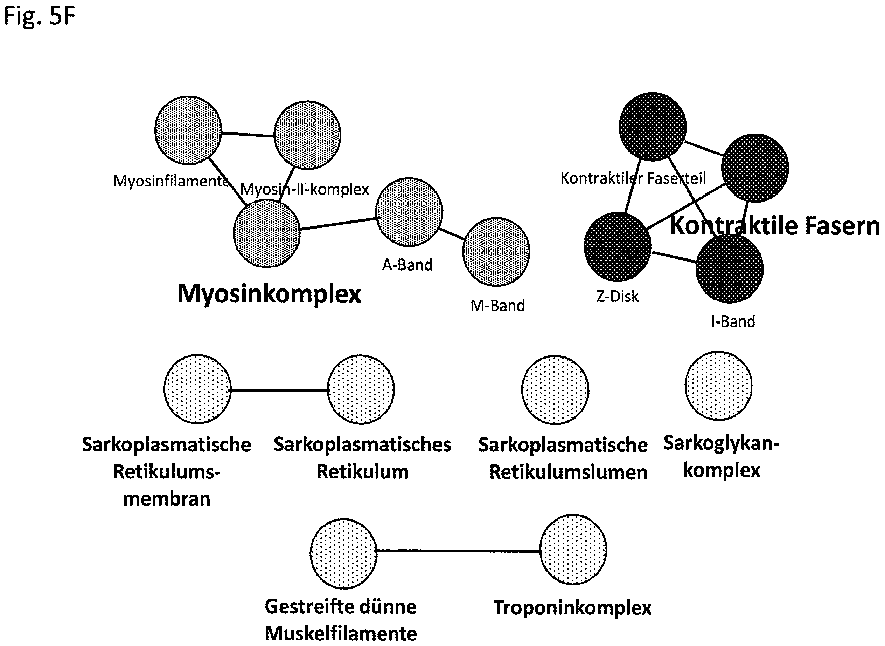

Zum Abschluss wurde der globale Status des Transkriptoms der iSABs mittels RNA-Seq-Analysen mit dem Ziel ermittelt, neuartige Faktoren und/oder Marker zu identifizieren, die für die Schrittmacher-Erzeugung von Bedeutung sind. Über diese Analyse wurden im Vergleich zu den Kontrollen in den iSABs 220 signifikant hochregulierte Gene identifiziert. Darunter waren Myh6 (33-fach hochreguliert), SERCA2 (20-fach hochreguliert), Ryr2 (39-fach hochreguliert) und Kcnj5 (45-fach hochreguliert). Insgesamt konnten die hochregulierten Gene 82 Gen-Ontologien zugeordnet werden, die biologische Prozesse beschreiben (Abb. 5A). Von diesen konnte die überwiegende Anzahl in Gruppen eingeteilt werden, die (äußerst) eng mit Herz- und Muskelfunktionen und der Herzentwicklung zusammenhängen (Abb. 5B). Das zugrundeliegende Netzwerk der Gen-Ontologien ist in Abb. 5C dargestellt. Bei der Adressierung von "zellulären Komponenten" fallen die 220 Gene in 34 Ontologien (Abb. 5D). Desgleichen könnten sie in Gruppen eingeteilt werden, die in hohem Maße mit für kontraktile Zellen typischen Strukturen assoziiert sind (Abb. 5E). Das zugrundeliegende Netzwerk der Gen-Ontologien ist in Abb. 5F dargestellt. Interessanterweise waren fast zwölffach mehr Gene (>2500) in den iSABs signifikant herunterreguliert, was möglicherweise mit der Rolle von TBX3 als transkriptioneller Repressor korreliert. Diese Gene hingen jedoch mehr mit Ontologien zusammen, die für sog. Housekeeping-Prozesse relevant sind, wie etwa die Signalkaskaden G-Protein-gekoppelter Rezeptoren und die Mitochondrienfunktion.

### 4. Diskussion

Die Kapazität zur *de novo* Produktion hoch angereicherter, aus Stammzellen gewonnener Populationen kardialer Schrittmacherzellen, die alle funktionellen Parameter reifer Sinusknotenzellen in sich tragen, ist von großem Interesse für zukünftige zellbasierte Therapien. Dies kann mit dazu beitragen, den ordnungsgemäßen Herzrhythmus im Sinne eines "biologischen Schrittmachers" wieder herzustellen. Außerdem wird das *in vitro* Arzneimittel-Screening von der Verfügbarkeit derartiger gereinigter Knotenzellen profitieren. Pluripotente Stammzellen (PSCs) stehen im Fokus dieser Ziele, da für sie nachgewiesen wurde, dass aus ihnen jeder Zelltyp des Säugerorganismus entstehen kann, einschließlich von spontan schlagenden Kardiomyozyten mit den molekularen und funktionellen Eigenschaften, die für SA-Zellen/Schrittmacherzellen charakteristisch sind. [10][22][23][24][25][11]. Allerdings sind die Zellpopulationen in "EmbryoidBodies" typischerweise äußerst heterogen, was zwangsläufig dazu führt, dass verlässliche Selektions- und Isolationsstrategien erforderlich sind - dies trifft im Besonderen auf den sehr seltenen Typ der kardialen Sinusknotenzellen zu. In diesem Zusammenhang wurden aus murinen ES-Zellen abgeleitete Zellen beschrieben, die durch den Promotor des HCN4-Gens transkriptionell kontrolliertes EGFP in sich tragen, die EGFP, HCN4 sowie andere kardiale Marker in spontan schlagenden Arealen ko-exprimieren[26]. Allerdings wurden nach der durchflusszytometrischen Aufreinigung in der EGFP-positiven Fraktion nur noch selten spontane Aktionspotentiale beobachtet und interessanterweise waren die meisten Zellen positiv für Nestin, einem Marker von Neuronen [26]. Dies stimmt mit dem Wissen überein, dass HCN4 sowohl ein Marker für spontan aktive Kardiomyozyten als auch für Nervenzellen ist [27]. Infolgedessen wurde als Alternative dazu die Suche nach endogenen OberflächenMarkern zur Reinigung dieser sehr begehrten Zellen vorangetrieben. In diesem Zusammenhang ist vor kurzem berichtet worden, dass eine auf Alcam (CD166) basierende Reinigung den Gehalt an Knotenzellen verbessert. Wie aus der Anfärbung von HCN4 ersichtlich ist, übertraf die Anreicherung des erwünschten Zelltyps allerdings nicht einmal 10 % in den aus der ES-Zellkultur gewonnenen resultierenden Zellen. Von den Zellen wurde berichtet, dass sie in der Lage sind, bei *in vitro* kultivierten ventrikuläre Kardiomyozyten Kontraktionen zu stimulieren, allerdings wurden diese wichtigen Ergebnisse nicht in der Veröffentlichung vorgestellt [12].

Andere Ansätze versuchten, die Erhöhung der Ausbeute an Knotenzellen aus differenzierenden ESCs über die pharmakologische Verabreichung niedermolekularer Verbindungen zu erzielen. Obwohl gewisse Erfolge (3-6-fache Zunahme) berichtet wurden, ergab dies bei weitem nicht eine ausreichend hohe Anreicherung funktioneller Sinusknotenzell-Populationen [28]. Desgleichen fehlt bei einer Veröffentlichung über die Verabreichung der niedermolekularen Verbindung EBIO, die eine Zunahme des Anteils an Knotenzellen beschreibt, die ausführlichen Analysen auf der elektrophysiologischen und funktionellen Ebene [9].

Um dieses Problem zu bewältigen, wurde ein Protokoll entwickelt, das die Programmierung zur gezielten Differenzierung von ES-Zellen unter Verwendung von TBX3 als einem wichtigen Transkriptionsfaktor mit einer Myh6-gestützten Antibiotika-Selektion kombiniert [15]. Dies führte durchweg zu einer sehr effektiven Erzeugung von sinusknoten-artigen Schrittmacherzell-Aggregaten, die durch spontan kontrahierende Kardiomyozyten mit hoch synchronisierten Schlagfrequenzen von 300 bis 400 bpm gekennzeichnet sind, welche zum ersten Mal in die Nähe der Frequenzen in einem Mäuseherz kommen. Die meisten (>80 %) der Kardiomyozyten in diesen Zell-Clustern repräsentierten eindeutig den gewünschten Typ der Knotenzelle -solche Reinheitensind vorher bei weitem nicht erreicht worden [9][28][12]. Da die erzeugten Zellen alle anderen Eigenschaften für eine vollständige Funktionalität aufweisen, d. h., Protein-Expressionsmuster, elektrophysiologische und Ca²⁺-Signal-Parameter, wird vorgeschlagen, diese gentechnisch erzeugten Schrittmacher-Aggregate als "induzierte sinuatriale Zellkörper" (iSABs) zu bezeichnen. Darüber hinaus sind die erzeugten iSABs funktionell hinsichtlich ihrer Potenz, das Kammermyokard *ex vivo* stabil zu stimulieren. Weiterhin stellen dieRNA-Seq-gestützten Analysen die ersten globalen mRNS-Expressionsmuster von aus Stammzellen abgeleiteten Schrittmacherzellen als Basis zur Untersuchung neuartiger Faktor- und/oder Markerkombinationen als Werkzeuge für die Programmierung und Reinigung von Schrittmacherzellen zur Verfügung.

Bemerkenswert ist, dass das anfängliche Pilotexperiment mit der alleine auf TBX3-gestützten Programmierung lediglich zu einer Verdopplung der Anzahl funktioneller Schrittmacherzellen in den Aggregaten mit signifikant niedrigeren Schlagfrequenzen führte. Die Anreicherung konnte allerdings durch die Einführung des zusätzlichen Myh6-Promotorgestützten Schrittes der Antibiotikaselektion drastisch weiter erhöht werden. Die Grundüberlegung basierte auf einer kürzlichen Veröffentlichung, welche die Anreicherung von ventrikulären und nodalen Kardiomyozyten über eine Myh6-gestützte Selektion beschreibt [15][17][18], obwohl diese Methode ursprünglich für die ergebnisoffene Anreicherung von Kardiomyozyten-Subtypen gedacht war.

Auf der anderen Seite ist die Tatsache, dass TBX3 alleine nicht zu reinen Populationen von aus ESC abgeleiteten Knotenzellen führt, im Einklang mit seinem Unvermögen zu einer vollständigen direkten Reprogrammierung des ventrikulären Myokards zu Schrittmacherzellen zu führen [4]. Kürzlich wurde enthusiastisch von Tbx18, einem weiteren T-Box-Transkriptionsfaktor, berichtet, dass er die direkte Umwandlung des Arbeitsmyokards in Sinusknotenzellen ("iSAN") ermöglicht. Während Tbx18 in diesem speziellen Zusammenhang erfolgversprechender erscheint als Tbx3, bleiben dennoch Vorbehalte bestehen, wie etwa die sehr geringe Wirksamkeit und relativ langsame Schlagfrequenzen. Darüber hinaus wird dieser virale Ansatz *in vivo* nur schwer zu kontrollieren sein [5]. Im neuen Paper [5a] dieser Gruppe zeigt sich genau dies: Der virale Vektor war auch in Lunge und Milz vorzufinden und auch im Herzen ergab sich kein klar fokussiertes Areal. Zudem konnte im Großtiermodell *in vivo* die Schrittmacherzellfunktion nur für zwei Wochen aufrechterhalten werden und verlor sich danach wieder. Zudem ist bisher unklar, ob es durch die adenovirale Infektion im Herzen zu Inflammation und/oder Arrhythmien kommen könnte.

Der erfinderische Ansatz unterscheidet sich ebenfalls von vorhergehenden Veröffentlichungen, die lediglich durch die Manipulation terminaler Effektormoleküle, die der sarkolemmalen Elektrophysiologie zugrunde liegen, biologische Schrittmacher simulieren, anstatt *de novo* vollständig funktionelle Knotenzellen zu erzeugen [29][30]. Im Gegensatz dazu führt das erfindungsgemäße Protokoll zu Zellen, die nicht nur elektrische Oszillationen, sondern auch die subtilen elektrophysiologischen und Ca²⁺-Signal-Eigenschaften und markanten morphologischen Merkmale nativer Schrittmacherzellen aufweisen. Daher repräsentiert die erfindungsgemäße Methode eine Basis für zukünftige Alternativen zu elektronischen Schrittmachern.

Obwohl die hier erzeugten Schrittmacherzellen ein synchronisiertes und schnell schlagendes Synzytium bilden und in der Lage sind, sich elektrisch mit ventrikulären Schnittkulturen *ex vivo* zu verbinden und in diesen Kontraktionen zu stimulieren, muss jedoch ihr Potential, sich intrakardial zu verankern und mit physiologischen Schlagraten Kontraktionen zu stimulieren, noch analysiert werden. Die Übertragbarkeit des Protokolls auf humane pluripotente Stammzellen (d. h., hESCs, hiPSCs) [31][32][33][34][35][36] ist ohne weiteres möglich. Dies kann letztendlich den Weg zur Verfügbarkeit von zellbasierten biologischen Schrittmachern ebnen, was eine große Bedeutung für klinische Anwendungen sowie für das Medikamenten-Screening *in vitro* besitzt.

### Material und Methoden

### qRT-PCR

Die quantitative Real-Time-PCR wurde unter Verwendung mit durch das Rneasy-Kit (Qiagen) isolierter RNS durchgeführt. Die Erststrang-cDNS wurde von 2 µgRNS mit AMV reverser Transkriptase (Amersham) und Random Hexamer Primer bei 37°C durchgeführt. Die Real-Time PCR wurde mit einem iCycler und dem MyiQDetection System (Biorad) unter Verwendung des IQ Syber Green Super Mix Kits (Biorad) durchgeführt. Die Primer wurden mit der DNS-Star Software konstruiert und die Spezifität jedes Primerpaares mittels Agarosegel-Elektrophorese bestätigt. Die Annealing-Temperatur betrug 57°C für alle Primerpaare und die amplifizierten murinen cDNS-Fragmente entsprechen den bp 812-934 der mGAPDH und den bp 287-429 von humanem TBX3 (hTbx3). Alle Proben wurden als Duplikate analysiert und die Gesamt-RNS, die aus undifferenzierten und differenzierten ES-Zellen sowie aus murinen Herzen vereinigt wurde, wurde als Kontrolle und zur Erstellung einer Standardkurve für die analysierten Marker verwendet. Als Negativkontrolle wurde die Gesamt-RNS von jeder Probe ohne reverse Transkriptaseeingesetzt. In Abwesenheit der reversen Transkriptase wurde nach 40 PCR-Zyklen kein Signal erhalten, was anzeigt, dass alle Proben frei von kontaminierender DNS waren. Darüber hinaus wurde auch kein Signal erhalten, wenn reverse Transkriptase ohne das RNS-Template zugesetzt wurde, was anzeigt, dass keine Kontamination durch exogene RNS oder DNS vorhanden war. Die Standardkurve für alle Gene zeigte eine Zunahme von einem Schwellenzyklus für jede Halbierung der Templatekonzentration. Die Evaluation der relativen Genexpressionsstärke wurde auf der Basis der ΔCT-Methode durchgeführt. Faktoren von Änderungen der relativen Stärke der mRNS-Expression wurden unter Verwendung von GAPDH als Referenzgen berechnet, wobei der Expressionswert in den Kontrollzellen mit 1 definiert wurde.

### Patch-Clamp-Technik

Spontane Aktionspotentiale und -ströme wurden von kontrahierenden Kardiomyozyten bei 37°C in der perforierten Patch-Konfiguration unter Verwendung eines MultiClamp 700B - Verstärkers und pClamp10-Software (Molecular Devices, Union City, USA) aufgezeichnet. Die offline-Datenanalyse wurde mit Clampfit-Software (Molecular Devices, Union City, USA) oder mittels Origin 6.0-Software (Microcal, Northampton, USA) durchgeführt. Patch-Pipetten wurden aus Borosilicatglas gezogen, Hitze-poliert und wiesen einen Widerstand von 2-5 MΩ bei Füllung mit Intrazellularlösung, die 10 mM NaCl 130 mM Kaliumaspartat, 0,04 mM CaCl₂, 3 mM Mg-ATP, 10 mM HEPES und 200 µg/ml Amphotericin B, pH-Wert eingestellt auf 7,2 mit KOH, enthielt, auf. Die extrazelluläre (Bad) Lösung enthielt: 140 mM NaCl, 5,4 mM KCl, 1 mM MgCl₂, 1,8 mM CaCl₂, 5 mM HEPES, 5,5 mM Glukose, der pH-Wert wurde eingestellt auf 7,4 mit NaOH. Für eine bessere Aufzeichnung des I_{f} wurden der Badlösung teilweise 2 mM BaCl₂ und 0,3 mM CdCl₂ zugesetzt, um I_{Kl} und I_{Ca} zu blockieren. I_{f} wurde durch schrittweise Veränderung ausgehend von einem Haltepotential von -40 mV zu Testpotentialen zwischen -130 mV und +20 mV gemessen. Die Stromamplitude nach 3 Sekunden während des -130 mV-Pulses wurde durch die Zellkapazitivität geteilt, um die Strom (I_{f})-Dichte zu bestimmen. Zur Bestimmung der I_{f} Aktivierungskinetiken wurde die Zeitkonstante der Aktivierung (τ) durch Anfitten der Stromspur des -130 mV-Schritts nach der initialen Verzögerung mit der Summe zweier Exponentialfunktionen y = A₁e^{(-x/τ}₁⁾ + A₂e^{(-x/τ}₂⁾ erhalten, wobei τ₁ und τ₂ schnelle bzw. langsame Zeitkonstkanten der Aktivierung sind; τ₁ wird demzufolge als τ bezeichnet, da die langsame Komponente (A₂) der HCN-Kanalaktivierung im Allgemeinen <10% der Stromamplitude beträgt. Isoproterenol oder Carbachol (Sigma, Taufkirchen, Deutschland) wurden direkt in der Badlösung am Tag des Experiments aufgelöst und den Zellen mittels eines schnellen Austausch-Superfusions-Systems verabreicht. APs wurden mit einer 10 kHz Abtastrate aufgezeichnet. Die Analysen wurden an den originalen Spuren durchgeführt. Die Neigung/Steigung des linearen Fits der Distanz vom MDP zur Potentialschwelle ist der DDR; Die AP-Dauer ist die Zeit vom Schwellenpotential zum MDP.

### Ca²⁺-Imaging

Alle Ca²⁺-Transienten wurde mittels Fluoreszenzbildgebungs-Mikroskopie (Visitron Systems) gemessen und mit VisiVIEW®Imaging-Software analysiert. Die Ca²⁺-Signale wurden bei einer Emissionswellenlänge von 525/50 nm mit einer Anregungswellenlänge von 470/40 nm (50 ms) unter Verwendung einer gekühlten CCD-Digitalkamera in einem 4*4 Binning-Modus aufgezeichnet. Die iSABs und aCaBs wurden mit 2,5 µM Fluo-4/AM in Differenzierungsmedium bei 37°C für 30 min beladen. nach einem zweiten Mediumwechsel wurden die Messungen bei einer Temperatur von 28°C durchgeführt. Die Ca²⁺-Transienten in ESCs sind als Spitzenwert der Fluoreszenzintensität (F) normalisiert auf die minimale Fluoreszenzintensität (Fo) während der analysierten Zeit angegeben.

Spontane Ca²⁺-Transienten wurden durch die Inhibierung der HCN-Kanäle mit 5 µM ZD 7288 (Sigma-Aldrich), die Inhibierung der spannungsabhängigen T-Typ-Ca²⁺-Kanäle mit 1 µM Mibefradil (Sigma-Aldrich) und die Inhibierung der spannungsabhängigen L-Typ-Ca²⁺-Kanäle mit 1 µM Nifedipin (Sigma-Aldrich) beeinflusst. Für die Experimente zur Blockierung des sarkolemmalen Ca²⁺-Transports wurde das Kulturmedium ersetzt durch eine Tyrode-Lösung in einer Zusammensetzung von (in mM): LiCI 140, KCl 6, MgCl 1, Glukose 10, EGTA 1, HEPES 5, pH 7,4 (eingestellt mit KOH). Der Einfluss der Ca²⁺-Speicher auf die Ca²⁺-Transienten wurde durch die Zugabe von Koffein (10 mM, Sigma-Aldrich), um die Ryanodinrezeptor-gekoppelten SR-Ca²⁺-Kanäle zu öffnen, und von Thapsigargin (2 µM, Sigma-Aldrich), um die SERCA zu inhibieren, untersucht. ZD 7288, Nifedipinuand Fluo-4/AM wurden in DMSO (Endkonzentration <0,1 %) und Thapsigargin in Ethanol (Endkonzentration 0,2 %) gelöst. Mibefradil und Koffein wurden in H₂O gelöst.

### Schnittpräparation und Kultur

Die Versuchsprotokolle für die Tierexperimente wurden durch die zuständige Bayrische Regierungsbehörde genehmigt und gemäß dem *Guide forthe Care andUseof Laboratory Animals* (National Institutes ofHealth, Veröffentlichung Nr. 85-23, überarbeitet 1996) durchgeführt. Herzen adulter Mäuse beider Geschlechter wurden rasch entfernt und in eine eiskalte modifizierte Tyrode-Lösung überführt (Zusammensetzung in mM): NaCl 136, KCl 5,4, MgCl₂ 1, CaCl₂ 0,9, NaH₂PO₄ 0,33, Glukose 10, 2,3-Butandion-monoxim 30, HEPES 5, pH 7,4 (eingestellt mit NaOH). Die Ventrikel wurden von den Arterien, Klappen und Gefäßen befreit und in einem 4 % Low-Melt Agarosegel, das in der modifizierten Tyrodelösung ohne Glukose gelöst war, eingebettet. Der Agaroseblock mit dem Herzen wurde auf den Probenhalter des Vibratoms (VT1200S, Leica) geklebt und schnell mit eiskalter Tyrodelösung überdeckt. Die Herzen wurden parallel zur Klappenebene in 300 µm dicke Gewebeschnitte mit Stahlklingen (Wilkinson) geschnitten, wodurch ringförmige Schnitte des ventrikulären Myokards erhalten wurden.

Nach einer Inkubation für 30 min in eiskalter Tyrodelösung wurden die Schnitte auf eine Biopore™ Membran von Gewebekultureinsätzen (Millicell, Millipore) für die Kultivierung an einer Luft-Medium-Grenzfläche übertragen. Die Gewebekultureinsätze wurden in PetriSchalen gesetzt, die Kulturmedium M199, angereichert mit 1% Insulin-Transferrin-Selenium (Gibco) und 1% Penicillin/Streptomycin (Sigma), enthielten. Die Schnitte wurden in einem Inkubator (37°C, 5 % CO₂) bis zur Kokultur kultiviert. Zur routinemäßigen Bestimmung der Lebensfähigkeit der Schnitte wurden diese mit Thiazolylblau-Tetrazoliumbromid (MTT, 0,5 mg/ml, Sigma) für 40 min bei 37°C inkubiert und unter einem Lichtmikroskop analysiert.

### Kokulturvon iSABs mit Herzschnitten

iSABs wurden mit CalceinRed™ (AAT Bioquest) zur Beobachtung der lebenden Zellen markiert. Die iSABs wurden dazu mit 10 mg/ml CalceinRed™/AM in Differenzierungsmedium für 30 min bei 37°C in dem Inkubator inkubiert. Nach Zentrifugation (1000 rpm, 5 min) und Resuspension in Differenzierungsmedium, wurden die iSABs mit einer Pipette unter dem Mikroskop auf den murinen Herzschnitten aufgetragen. iSABs und Herzschnitte wurden in dem Inkubator (37°C, 5 % CO₂) kokultiviert. Die Schlagfrequenzen der iSABs und der Schnittbereiche wurden unter Verwendung einer Sony NEX-5N Kamera täglich aufgezeichnet. Die Kopplung zwischen iSABs und den Schnitten wurde durch die Übertragung von CalceinRed™ von den iSABs in die Zellen der Schnitte mittels Fluoreszenzbildgebung (Ex/Em 646/659 nm, Visitron Systems) nachgewiesen. Die Kontaktbereiche der iSABs mit den Schnitten wurden täglich aufgezeichnet und die Verteilung der CalceinRed™-Fluoreszenz visuell analysiert.

### Ca²⁺ Imaging in Kontaktbereichen der iSABs mit den Schnitten

Die Schnitte mit iSABs wurden mit 5 µM Fluo-4/AM (Invitrogen) in M199, angereichert mit 1% Insulin-Transferrin-Selenium (Gibco) und 1 % Penicillin/Streptomycin (Sigma) bei 37°C für 30 min beladen. Die Ca²⁺-Signale wurden durch Fluoreszenzbildgebungs-Mikroskopie (Visitron Systems) bei einer Emissionswellenlänge von 525/50 nm und mit einer Anregungswellenlänge von 470/40 nm (Expositionszeit 30 ms) unter Verwendung einer gekühlten CCD-Digitalkamera in einem 4*4 Binning-Modus aufgezeichnet. Alle Ca²⁺-Transienten wurden mit VisiVIEW® Imaging Software analysiert und als Spitzenwert der Fluoreszenzintensität (F) angegeben, normalisiert auf die minimale Fluoreszenzintensität (Fo) während der Analysezeit (20 s) in den entsprechenden Bereichen.

### Zellkultur

Die von der murinen Zelllinie GSES [37] abgeleiteten ES-Zelllinien wurden in DMEM-Medium mit hoch konzentrierter Glukose und stabilem Glutamin (GIBCO), das 10% FBS Superior (Biochrom), 100 µM nichtessentielle Aminosäuren (GIBCO), 1% Penicillin/Streptomycin (GIBCO) und 100 µM β-Mercaptoethanol (Sigma) enthält, in Anwesenheit von 1000 U/mLLeukemia-Inhibitory-Factor (LIF, Milllipore) wachsen lassen. Die Differenzierung wurde gemäß den Standardprotokollen in Iscove's Basalmedium (Biochrom) durchgeführt, das 10% FBS (Biochrom), 100 µM nichtessentielle Aminosäuren (GIBCO), 1% Penicillin/Streptomycin (GIBCO) und 450 µM 1-Thioglycerol enthält. [13]. Die Passage der Zellen wurde mit Trypsin/EDTA (GIBCO) bei 70% Konfluenz durchgeführt, die normalerweise nach zwei bis drei Tagen erreicht wurde. Die Differenzierung wurde gewöhnlich 3 bis 4 Passagen nach dem Auftauen der Zellen durchgeführt. Die Zellen wurden mit 15 µg Plasmid-DNS unter Verwendung von JetPEI (Peqlab) und nachfolgender Selektion mit 10 µg/mLBlasticidin (Invivogen) oder 250 µg/mLHygromycin (Invitrogen) in 10 cm Gewebekulturschalen transformiert. Stabile Klone wurden manuell ausgewählt, danach kultiviert und mittels qRT-PCR und Differenzierungs-Assays getestet [13]. Zur Induktion der spontanen Bildung von aCaBs und iSABs wurden positive Klone wie kürzlich beschrieben [15] zur Anreicherung von Kardiomyozyten mit Antibiotika behandelt. Die für die nachfolgenden physiologischen Analysen benötigten Einzelzellen wurden wie beschrieben enzymatisch isoliert [14][16]. Zur Absicherung der erfolgreichen Erzeugung von aCaBs und iSABs wurden mögliche Kontaminationen mit Mykoplasmen regelmäßig zweimal wöchentlich mit dem PCR-basierten MycoSPY-Kit System (Biontex) kontrolliert.

### RNS-Sequenzierung

Für die Erzeugung einer Bibliothek und zur Sequenzierung wurde das Kulturmedium der kultivierten, adhärent wachsenden Zellen dekantiert, die Zellen gewaschen und direkt durch Zugabe von Lysepuffer lysiert. Von diesem Lysat wurde 1 µl für die cDNS-Synthese und Amplifikation mit dem SMARTer Kit (Clontech, Mountain ViewCA, USA) entsprechend den Herstellerangaben eingesetzt. Die cDNS-Synthese wurde durch Annealing eines polyAspezifischen Primers und Zugabe einer reversen Transkriptase mit terminaler Transferaseaktivität gestartet. Anschließend wurde die neu synthetisierte Erststrang-cDNS zuerst durch die terminale Transferase mit einem Homopolymer-Stretch und dann mittels Template-Switching mit einem spezifischen Amplifizierungs-Tag verlängert. Die resultierende doppelt markierte cDNS wurde mittels PCR amplifiziert, durch Ultraschallbehandlung (Bioruptor, Diagenode, Lüttich, Belgien; 25 Zyklen mit 30s an/30s aus) fragmentiert und in bar-kodierte Illumina-Sequenzbibliotheken unter Verwendung des NEBnext Ultra DNS Library Preparationkit (New England Biolabs, Ipswich MA, USA) konvertiert. Nach PCR-Anreicherung wurden die cDNA-Bibliotheken mit AmpureXP Magnet-Beads (Beckman-Coulter, Brea CA, USA) gereinigt und auf einem Bioanalyzer 2100 (Agilent, Santa Clara CA, USA) quantifiziert. Die cDNA-Bibliotheken wurden mit äquimolaren Mengen vereinigt und auf einem IlluminaGenomeAnalyzerllx im Single-Read-Modus mit einer Readlänge von 78 Nukleotiden und einem Umfang von 21 Millionen bis 32 Millionen Raw Reads pro Replikat sequenziert.

Für das nachfolgende Mapping und die Expressionsanalysen wurden die Reads der Illumina-Sequenzierung de-multiplexiert und mit dem mm9 Mausgenom mit dem Spliced-read Mapper TOPHAT (v1.4) [38] und einer Genomannotation (von iGenomes, http://cufflinks.cbcb.umd.edu/igenomes.html) für die Unterstützung des Nachweises der Exon-Exon-Grenzen gemappt. Die Zuordnung der Reads für jedes Gen wurden mit HTSeq (http://www-huber.embl.de/users/anders/HTSeq/) erhalten und mit dem DESeq R-Package normalisiert [39]. Die differentielle Expression wurde mittels DESeq mit paarweisem Vergleich von jeder Gruppe mit drei biologischen Replikaten und mit einer auf 0,05 eingestellten False Discovery Rate untersucht. Alle Sequenz- und Daten-Analyseschritte wurden auf einem lokalen Server der Galaxy-Plattform durchgeführt [40].

### Beschreibung der Abbildungen

**Abb. 1****: Funktionalität der TBX3-Überexpressionskonstrukte in ES-Zellen**
   (A) 20 unabhängige Zellklone, die mit dem die humane TBX3-cDNS enthaltenden Überexpressionskonstrukt stabiltransfiziert sind. Die Überexpressions-Level wurden mittels qRT-PCR analysiert. Für die weitere Analyse wurden vier repräsentative Klone ausgewählt (Daten sind als Mittelwerte ± SD angegeben; n = 2). -(B) Die Immunfärbungen von überexprimiertem TBX3 und Aktin in den vier ausgewählten Klonen bestätigt die Überexpressionslevel von TBX3. (C) FACS-Analysen von Oct-4/Pou5f1 und Sox2 zeigen keinen Einfluss der TBX3-Überexpression auf die Pluripotenz unter der Zugabe von LIF (Daten sind als Mittelwerte ± SEM angegeben; n = 5).
**Abb. 2****: Überwiegendes Auftreten schrittmacherartiger Kardiomyozyten in Tbx3-differenzierten ES-Zellen**
   (A) Zunahme der spontanen Schlagaktivität in unabhängigen TBX3-Klonen und in ES-Kontrollzellen (GSES) (Daten sind als Mittelwerte ± SEM angegeben, n> 100). Die Kontrolle und die vier Klone Klon #3, Klon #7, Klon #15 und Klon #19 sind jeweils in dieser Reihenfolge von rechts nach links gezeigt, beginnend jeweils bei der in weiß gezeigten Kontrolle. (B) Konfokalanalyse der Myh6-Expression in Kontroll- und TBX3 überexprimierenden Kardiomyozyten. Gegenfärbung von Aktin und Zellkernen . Maßstab: 10 µm. (C) Verteilung der Kardiomyozyten-Subtypen und Ventr. - ventrikelartige (23,1 %); Atr. - vorhofartige (8 %); Pace. - Sinuatrialknoten-artige (38,5 %); Interm. - Intermediärer/früher Typ (38,8 %). waagrechter Balken: 100 ms; senkrechter Balken: 20 mV. (D) HCN4-exprimierende Zellen weisen signifikant erhöht TBX3-Klone an Tag 18 auf (Daten sind als Mittelwerte ± SD angegeben; n = 5).
**Abb. 3****: Physiologische Parameter der durch kombinierte Tbx3-Differenzierung und Myh6-Promotor-Selektion erhaltenen schrittmacherartigen Kardiomyozyten**
   (A) Zeitverlauf der Schlagfrequenzen nach unterschiedlichen Behandlungsregimes von Myh6-Neomycin-Kontrollen und Myh6-TBX3-Zellen.Die höchsten Schlagfrequenzen (300 - 400 bpm) werden über eine Kombination aus gezielter TBX3-Programmierung, Antibiotikaselektion und einem zusätzlichen Dissoziationsschritt erreicht (Daten sind als Mittelwerte ± SD angegeben; n≥ 5). (B) Expression von HCN4 (linker Bereich), Cx45 (mittlerer Bereich) und Cx30.2 (rechter Bereich) in iSABs. Gegenfärbung von Aktin und Zellkernen. Maßstab: 10 µm. (C) Typische langgestreckte Zellform von Sinusknotenzellen in den durch iSAB-Ausplattierung erhaltenen synchronisierten Zellschichten. Maßstab: 10 µm. (D) Verteilung der Schrittmacherzellen, wie sie aus Einzelzell-Patch-Clamp- und Funny-Kanal-Messung ersichtlich ist: es wurden mehr als 81 % schrittmacherartige Zellen erhalten. Von diesen waren 19 % unreife Schrittmacherzellen, während die übrigen reife Schrittmacherzellen waren; n = 65, (E) Frequenzen spontaner Ca²⁺-Transienten in Myh6-TBX3-Zellen nehmen nach Inhibierung der HCN-Kanäle durch ZD7288 signifikant ab (Daten sind als Mittelwerte ± SEM angegeben; n ≥ 7). (F) Die Frequenzen spontaner Ca²⁺-Transienten in Myh6-TBX3-Zellen nehmen nach Inhibierung von T-Typ- und L-Typ-Ca²⁺-Kanälen durch Mibefradil und Nifedipin ab (Daten sind als Mittelwerte ± SEM angegeben; n > 12). (G) Spontane Ca²⁺-Transienten vor und in Anwesenheit von 10 mM Koffein in aus iSABs und aCaBs abgeleiteten Zellen. Die Amplitude des Koffein-induzierten Peaks in den erstgenannten Zellen ist mit den Maxima der spontanen Ca²⁺-Transienten vergleichbar. n ≥ 8. (H) Blockade der Ca²⁺-Aufnahme in das SR durch Thapsigargin führt im Gegensatz zu den aus aCaBs abgeleiteten Zellen in aus iSABs abgeleiteten Zellen zu erhöhten diastolischen Ca²⁺-Spiegeln. n ≥ 11. (I) Ca²⁺-Transienten vor und nach Blockade des Na⁺/Ca²⁺-Austauschers und der sarkolemmalen Ca²⁺-Kanäle: in aus iSABs abgeleiteten Zellen wird im Gegensatz zu den aus EBs abgeleiteten Kontrollzellen ein großer Koffein-Peak beobachtet. n > 16. (J) Ca²⁺-Transienten vor und nach Inhibierung der Ca²⁺-Aufnahme: Inhibierung von Na⁺/Ca²⁺-Austauscher plus SERCA-Inhibierung verursacht im Gegensatz zu den aus aCaBs abgeleiteten Zellen einen schnellen Anstieg desintrazellulären Ca²⁺ in aus iSABs abgeleiteten Zellen. n > 24. Zusätzlich wurde in beiden Fällen ein kleiner Koffein-Peak beobachtet. (K) Die Analyse des SR-Ca²⁺-Ausstroms, die für als Schrittmacher aktive Zellen charakteristisch ist, zeigt in aus iSABs abgeleiteten Zellen im Vergleich zu aCaBs eine erhöhte Rate der Akkumulation von intrazellulärem Ca²⁺. Blockade des Na⁺/Ca²⁺-Austauschers plus Inhibierung von SERCA bewirkte eine intrazelluläre Ca²⁺-Akkumulation vergleichbar mit der Verringerung des Koffein-Peaks in aus iSABs abgeleiteten Zellen. Kein Unterschied in der Rate der Akkumulation von zytosolischem Ca²⁺ und Amplitude des Koffein-Peaks in aus aCaBs abgeleiteten Zellen. Mittelwert ± SEM; #p<0.05 vs. ohne SERCA-Inhibierung; *p<0.05 vs. Kontrollen.
**Abb. 4****: Funktionalität von iSABs im *Ex-vivo-*Modell kultivierter murinerVentrikelschnitte**
   (A) Für fünf Tage kultivierte Ventrikelschnitte (linker Bereich) und MTT-Anfärbung zur Überprüfung der Vitalität (rechter Bereich). (B) Identifizierung der auf einem Schnitt ausgesäten DiI-markierten iSABs (rechter Bereich: overlay) (C) Prozentuale Verteilung von iSAB-, aCaB- und nicht-besäten Schnitten, die mindestens einen kontrahierenden Bereich über die Zeit enthalten. Die spontane Aktivität nimmt nach Tag 2 ab, während die Aktivität in den mit iSAB ausgesäten Schnitten in hohem Maße erhalten bleibt (n ≥ 15). (D) Durchschnittliche Anzahl der aktiven Regionen je iSAB-, aCaB- und nicht-besäte Schnitte über die Zeit (Daten sind als Mittelwerte ± SEM angegeben (MW SEM); n ≥ 15). (E) Zunahme der Schlagfrequenzen von mit iSABs besäten Schnitten von Tag 1 bis Tag 4 (Daten sind als Mittelwerte ± SEM angegeben ((MW SEM); n ≥ 16). (F) Übertragung von Calcein-Farbstoff aus einem iSAB auf den Empfängerschnitt über die Zeit [Maßstab: 200 µm; "h": Stunde(n); "d": Tag(e)]. (G) Die Stimulation von Schnittregionen (Pfeile) in der näheren Umgebung eines iSAB (Pfeil) wird durch hoch synchrone Ca²⁺-Transienten begleitet.
**Abb. 5****: Durch RNS-Seq ermittelter Transkriptom-Status von iSABs**
   Ontologie-Bezeichnungen, die dem Fachmann nur in englischer Sprache gebräuchlich sind, wurden hier in Englisch belassen.
   (A) 82 Genontologien, die biologische Prozesse beschreiben, umfassen 220 hochregulierte Gene in iSABs vs. Kontrollen. (B) Cluster von Ontologie-Gruppen mit Bezug zu Herz-/Muskel-Funktion und Herzentwicklung dominieren. (C) Zusammengefasstes Netzwerk der Gen-Ontologien, die sich auf biologische Prozesse beziehen. (D) 34 Gen-Ontologien, die zelluläre Komponenten beschreiben, umfassen die 220 hochregulierten Gene in iSABs vs. Kontrollen. (E) Cluster von Ontologie-Gruppen mit Bezug zu Strukturen, die für kontrahierende Zellen typisch sind, dominieren. (F) Zusammengefasstes Netzwerk der Genontologien, die sich auf zelluläre Komponenten beziehen.

### 7. Zusätzliches Informationsmaterial

**Tabelle 1: Aktionspotentialparameter der aus Action Tbx3-programmierten Zellen erhaltenen Kardiomyozyten.**
   Die Daten sind der Mittelwert ± SD (Standardabweichung, standard deviation).
   In der Tabelle werden Kommata (,) in den Zahlenangaben durch Punkte (.) wiedergegeben.

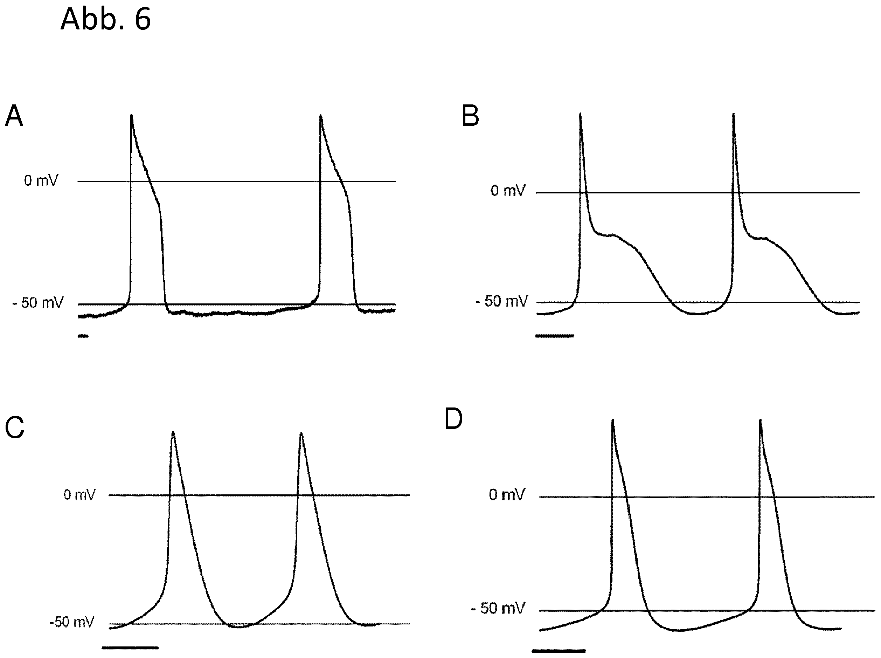

   **Abb. 6****: Repräsentative Aktionspotentiale erhalten von Tbx3-programierten Zellen**
   (A, B) Myocardial-Typen mit (A) ventrikular-artigen und (B) atrial-artigen Aktionspotentialen. (C, D) SChrittmacher-Typus-Aktionspotentiale mit (C) reifen sinoatrial-artigen Aktionspotentialen und (D) leicht unreifen Schrittmacher-artigen Zellen, die als Intermediat-Typus-Zellen des murinen embryonalen Herzen bezeichnet werden. Horizontale Zeitachse: 100 ms.
**Tabelle 2: Aktionspotential-Parameter von Kadromyozyten, die aus iSABs erhalten wurden.**
   Die Daten sind der Mittelwert ± SD.
   In der Tabelle werden Kommata (,) in den Zahlenangaben durch Punkte (.) wiedergegeben.

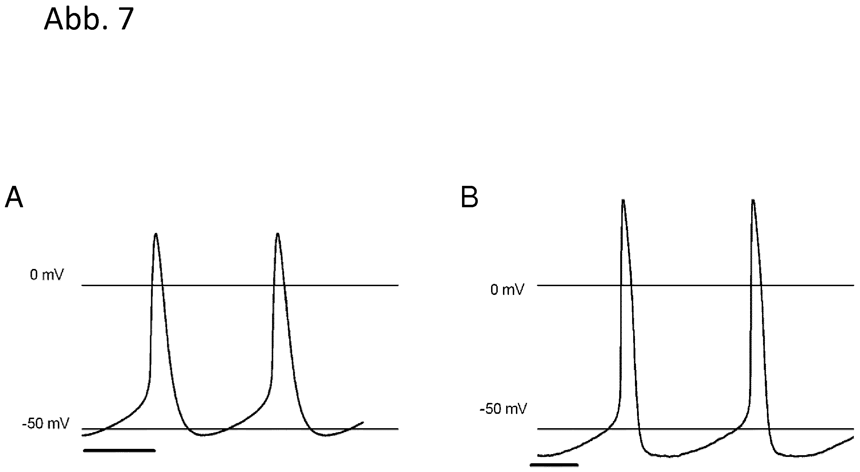

   **Abb. 7****: Repräsentative Aktionspotentiale erhalten aus iSAB-Kardiomyozyten.**
      (A) Schrittmacher-Typus Aktionspotentiale ähnelnd Aktionspotentialen, die von ausgewachsenen murinen sinoatrialen Knotenzellen generiert werden. (B) Aktionspontentiale generiert durch leicht unreife Schrittmacher-artige Zellen. Horizontale Zeitachse: 100 ms.

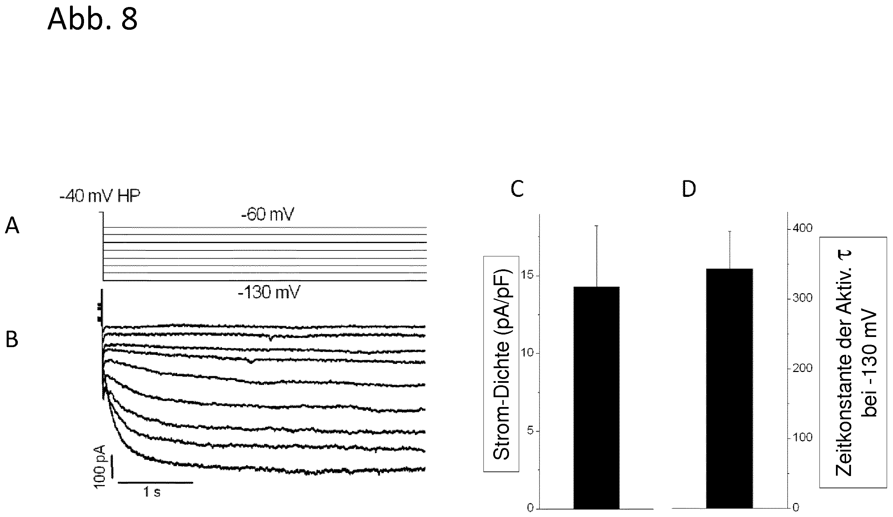

   **Abb. 8****: I_{f} -Aufzeichnungen aus isolierten iSAB-Zellen.**
      (A) Patch-clamp-Protokoll; Spannung angelegt zum Hervorrufen des durch Hyperpolarisation aktivierten Stromes. (B) Beispiel des I_{f} Stromes, aufgezeichnet von einer isolierten aus einem iSAB-stammenden Zelle. (C) Stromdichte und (D) Zeitkonstante der Aktivierung bei-130 mV, zeigend eine robust I_{f}-Expression mit langsamen Aktivierungskinetiken typisch für den HCN4-Kanal-Subtypus und für reife sinoatriale Knoten I_{f}. n= 17; Fehlerbalken: Mittelwert ± SD.

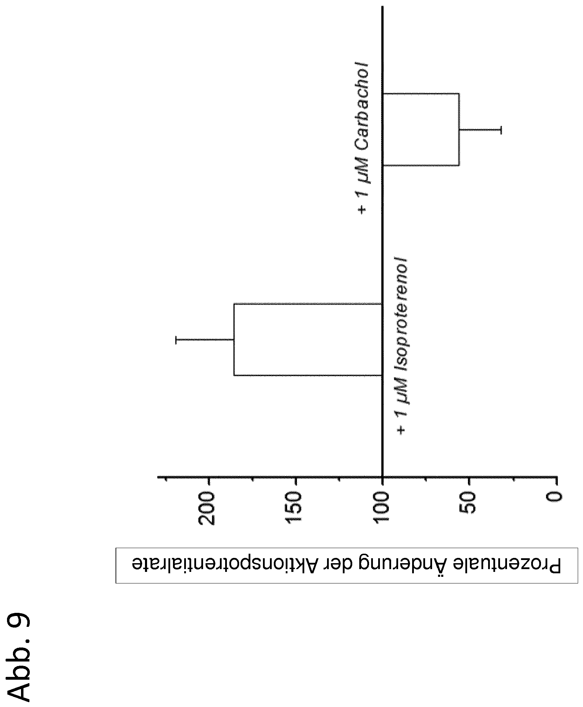

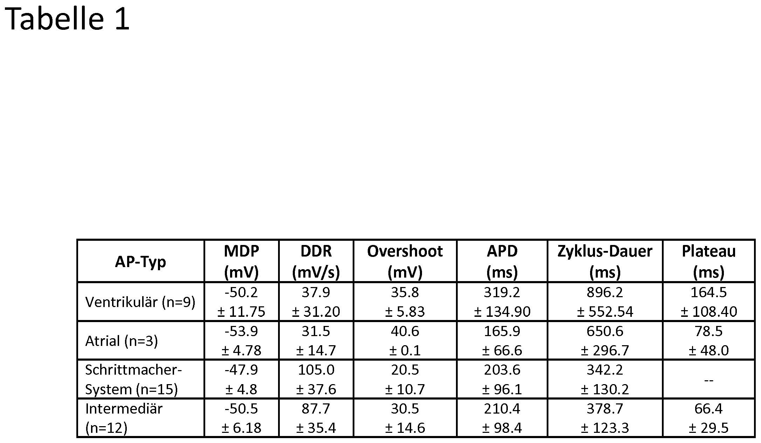

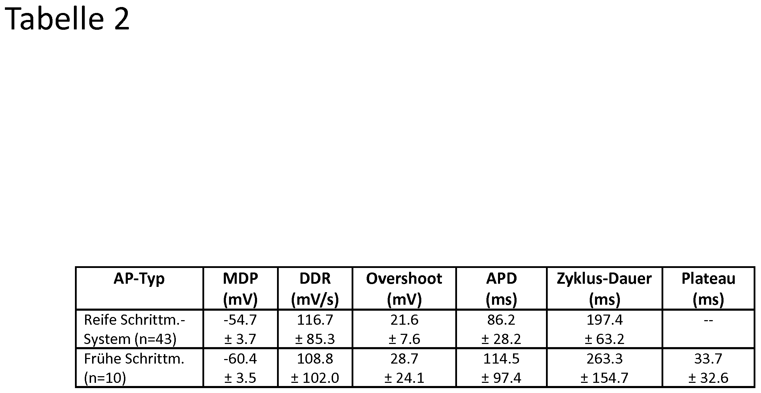

   **Abb. 9****: Modulation von Aktionspotential (AP)-Raten von isolierten Tbx3-programmierten und von iSAB-stammenden Zellen.**
      Reaktion auf β-adrenerge (Isoprotenerol) und muscarine (Carbachol) Stimulation führt zu typisch beschleunigten vs. verlangsamten AP-Raten. Von iSAB stammende Zellen zeigen eine deutlichere Antwort auf Isoprotenerol mit Schlagfrequenzen, die bis zu 560bpm.iSAB/Iso reichen: n=9; iSAB/Carb: n=5; Fehlerbalken: Mittelwert ± SD.

### Literatur

1. Rosen, M.R., Biological pacemaking: in our lifetime? Heart Rhythm., 2005. 2(4): S. 418-28.
2. Ishii, T.M., et al., Molecular characterization of the hyperpolarization-activated cation channel in rabbit heart sinoatrial node. J Biol Chem., 1999. 274(18): S. 12835-9.
3. Stieber, J., F. Hofmann, and A. Ludwig, Pacemaker channels and sinus node arrhythmia. Trends Cardiovasc Med, 2004. 14(1): S. 23-8.
4. Bakker, M.L., et al., T-box transcription factor TBX3 reprogrammes mature cardiac myocytes into pacemaker-like cells. Cardiovasc Res, 2012. 94(3): S. 439-49.
5. Kapoor, N., et al., Direct conversion of quiescent cardiomyocytes to pacemaker cells by expression of Tbx18. Nat Biotechnol, 2013. 31(1): S. 54-62.
5a. Hu, Y.-F., et al., Biological pacemaker created by minimally invasive somatic reprogramming in pigs with complete heart block. Sci Transl Med , 2014. 245(6): S. 1-10.
6. Wiese, C., et al., Formation of the sinus node head and differentiation of sinus node myocardium are independently regulated by Tbx18 and Tbx3. Circ Res, 2009. 104(3): S. 388-97.
7. Wobus, A.M., G. Wallukat, and J. Hescheler, Pluripotent mouse embryonic stem cells are able to differentiate into cardiomyocytes expressing chronotropic responses to adrenergic and cholinergic agents and Ca2+ channel blockers. Differentiation, 1991. 48(3): S. 173-82.
8. Kehat, I., et al., High-resolution electrophysiological assessment of human embryonic stem cell-derived cardiomyocytes: a novel in vitro model for the study of conduction. Circ Res, 2002. 91(8): S. 659-61.
9. Kleger, A., et al., Modulation of calcium-activated potassium channels induces cardiogenesis of pluripotent stem cells and enrichment of pacemaker-like cells. Circulation, 2010. 122(18): S. 1823-36.
10. Maltsev, V.A., et al., Cardiomyocytes differentiated in vitro from embryonic stem cells developmentally express cardiac-specific genes and ionic currents. Circ Res, 1994. 75(2): S. 233-44.
11. David, R. and W.M. Franz, From pluripotency to distinct cardiomyocyte subtypes. Physiology (Bethesda), 2012. 27(3): S. 119-29.
12. Scavone, A., et al., Embryonic stem cell-derived CD166+ precursors develop into fully functional sinoatrial-like cells. Circ Res, 2013. 113(4): S. 389-98.
13. David, R., et al., MesP1 drives vertebrate cardiovascular differentiation through Dkk-1-mediated blockade of Wnt-signalling. Nat Cell Biol., 2008. 10(3): S. 338-45. Epub 2008 Feb 24.
14. David, R., et al., Forward programming of pluripotent stem cells towards distinct cardiovascular cell types. Cardiovasc Res., 2009. 84(2): S. 263-72. Epub 2009 Jun 29.
15. Klug, M.G., et al., Genetically selected cardiomyocytes from differentiating embronic stem cells form stable intracardiac grafts. J Clin Invest, 1996. 98(1): S. 216-24.
16. David, R., et al., Selection of a common multipotent cardiovascular stem cell using the 3.4-kb MesP1 promoter fragment. Basic Res Cardiol, 2013. 108(1): S. 312.
17. Kensah, G., et al., Murine and human pluripotent stem cell-derived cardiac bodies form contractile myocardial tissue in vitro. Eur Heart J, 2012. 34(15): S. 1134-46.
18. Otsuji, T.G., et al., Progressive maturation in contracting cardiomyocytes derived from human embryonic stem cells: Qualitative effects on electrophysiological responses to drugs. Stem Cell Res, 2010. 4(3): S. 201-13.
19. Kreuzberg, M.M., et al., Functional properties of mouse connexin30.2 expressed in the conduction system of the heart. Circ Res, 2005. 96(11): S. 1169-77.
20. Verheijck, E.E., et al., Electrophysiological features of the mouse sinoatrial node in relation to connexin distribution. Cardiovasc Res, 2001. 52(1): S. 40-50.
21. Halbach, M., et al., Ventricular slices of adult mouse hearts--a new multicellular in vitro model for electrophysiological studies. Cell Physiol Biochem, 2006. 18(1-3): S. 1-8.
22. He, J.Q., et al., Human embryonic stem cells develop into multiple types of cardiac myocytes: action potential characterization. Circ Res, 2003. 93(1): S. 32-9.
23. Yanagi, K., et al., Hyperpolarization-activated cyclic nucleotide-gated channels and T-type calcium channels confer automaticity of embryonic stem cell-derived cardiomyocytes. Stem Cells, 2007. 25(11): S. 2712-9.
24. Barbuti, A., et al., Molecular composition and functional properties of f-channels in murine embryonic stem cell-derived pacemaker cells. J Mol Cell Cardiol, 2009. 46(3): S. 343-51.
25. Ma, J., et al., High purity human-induced pluripotent stem cell-derived cardiomyocytes: electrophysiological properties of action potentials and ionic currents. Am J Physiol Heart Circ Physiol, 2011. 301(5): S. H2006-17.
26. Morikawa, K., et al., Identification, isolation and characterization of HCN4-positive pacemaking cells derived from murine embryonic stem cells during cardiac differentiation. Pacing Clin Electrophysiol, 2010. 33(3): S. 290-303.
27. Garcia-Frigola, C., Y. Shi, and S.M. Evans, Expression of the hyperpolarization-activated cyclic nucleotide-gated cation channel HCN4 during mouse heart development. Gene Expr Patterns, 2003. 3(6): S. 777-83.
28. Wiese, C., et al., Differentiation induction of mouse embryonic stem cells into sinus node-like cells by suramin. Int J Cardiol, 2011. 147(1): S. 95-111.
29. Johns, D.C., et al., Adenovirus-mediated expression of a voltage-gated potassium channel in vitro (rat cardiac myocytes) and in vivo (rat liver). A novel strategy for modifying excitability. J Clin Invest, 1995. 96(2): S. 1152-8.
30. Nuss, H.B., E. Marban, and D.C. Johns, Overexpression of a human potassium channel suppresses cardiac hyperexcitability in rabbit ventricular myocytes. J Clin Invest, 1999. 103(6): S. 889-96.
31. Evans, M.J. and M.H. Kaufman, Establishment in culture of pluripotential cells from mouse embryos. Nature, 1981. 292(5819): S. 154-6.
32. Martin, G.R., Isolation of a pluripotent cell line from early mouse embryos cultured in medium conditioned by teratocarcinoma stem cells. Proc Natl Acad Sci U S A, 1981. 78(12): S. 7634-8.
33. Gerecht-Nir, S., B. Fishman, and J. Itskovitz-Eldor, Cardiovascular potential of embryonic stem cells. Anat Rec A Discov Mol Cell Evol Biol, 2004. 276(1): S. 58-65.
34. Takahashi, K. and S. Yamanaka, Induction of pluripotent stem cells from mouse embryonic and adult fibroblast cultures by defined factors. Cell., 2006. 126(4): S. 663-76. Epub 2006 Aug 10.
35. Takahashi, K., et al., Induction of pluripotent stem cells from adult human fibroblasts by defined factors. Cell., 2007. 131(5): S. 861-72.
36. Mauritz, C., et al., Generation of functional murine cardiac myocytes from induced pluripotent stem cells. Circulation., 2008. 118(5): S. 507-17. Epub 2008 Jul 14.
37. David, R., M. Groebner, and W.M. Franz, Magnetic cell sorting purification of differentiated embryonic stem cells stably expressing truncated human CD4 as surface marker. Stem Cells, 2005. 23(4): S. 477-82.
38. Trapnell, C., L. Pachter, and S.L. Salzberg, TopHat: discovering splice junctions with RNS-Seq. Bioinformatics, 2009. 25(9): S. 1105-11.
39. Anders, S. and W. Huber, Differential expression analysis for sequence count data. Genome Biol, 2010. 11(10): S. R106.
40. Goecks, J., A. Nekrutenko, and J. Taylor, Galaxy: a comprehensive approach for supporting accessible, reproducible, and transparent computational research in the life sciences. Genome Biol, 2010. 11(8): S. R86.

## Patentansprüche

1. Verfahren zur Erzeugung von Sinusknotenzellen aus Stammzellen, insbesondere pluripotenten Stammzellen, bei welchem eine Nukleinsäure in Stammzellen eingebracht wird, wodurch diese einen TBX3-Transkriptionsfaktor exprimieren, oder ein TBX3-Protein in die Stammzellen eingebracht wird,
**dadurch gekennzeichnet, dass**
zusätzlich ein Konstrukt zur Expression eines Antibiotikum-Resistenz-Gens, welches durch einen alpha-MHC(MYH6)-Promoter gesteuert wird, eingebracht wird und die resultierenden Stammzellen in Gegenwart des Antibiotikums differenziert werden.

2. Verfahren zur Erzeugung von Sinusknotenzellen aus Stammzellen nach Anspruch 1, **dadurch gekennzeichnet, dass** multipotente oder pluripotente, vorzugsweise pluripotente, Stammzellen eingesetzt werden.

3. Verfahren zur Erzeugung von Sinusknotenzellen aus Stammzellen nach Anspruch 1 oder 2, **dadurch gekennzeichnet, dass** nicht-humane embryonale Stammzellen oder nicht-humane induzierte Stammzellen oder humane induzierte Stammzellen oder parthenogenetische Stammzellen oder spermatogoniale Stammzellen eingesetzt werden, vorzugsweise nicht-humane embryonale Stammzellen oder nicht-humane induzierte Stammzellen oder humane induzierte Stammzellen.

4. Verfahren zur Erzeugung von Sinusknotenzellen aus Stammzellen nach einem oder mehreren der Ansprüche 1-3, **dadurch gekennzeichnet, dass** die Nukleinsäure ausgewählt ist aus TBX3-DNS, insbesondere TBX3-cDNS; TBX3-RNS, insbesondere TBX3-mRNS.

5. Verfahren zur Erzeugung von Sinusknotenzellen aus Stammzellen nach einem oder mehreren der Ansprüche 1-4, **dadurch gekennzeichnet, dass** die Einbringung derTBX3-Nukleinsäure mittels Vektor, insbesondere mittels (Über-)Expressionsvektor, erfolgt.

6. Verfahren zur Erzeugung von Sinusknotenzellen aus Stammzellen nach einem oder mehreren der Ansprüche 1-5, **dadurch gekennzeichnet, dass** das Antibiotikum-Resistenz-Gen ausgewählt ist aus Aminoglykosid-Antibiotikum-Resistenz-Gen, mehr bevorzugt aus Neomycin- und Puromycin-Resistenz-Gen, höchst bevorzugt Neomycin-Resistenz-Gen; und dass das Antibiotikum entsprechend ausgewählt ist aus Aminoglykosid-Antibiotikum, insbesondere aus Neomycin und Puromycin.

7. Verwendung von *in vitro* aus Stammzellen gemäß einem Verfahren nach einem oder mehreren der Ansprüche 1-6 erzeugten Sinusknotenzellen zur *in vitro*-Evaluation von Arzneimitteln.

8. Verwendung von *in vitro* aus Stammzellen gemäß einem Verfahren nach einem oder mehreren der Ansprüche 1-6 erzeugten Sinusknotenzellen zur *in vitro* Herzgewebekonstruktion und/oder Herzgewebezüchtung.

9. Verwendung von *in vitro* aus Stammzellen gemäß einem Verfahren nach einem oder mehreren der Ansprüche 1-6 erzeugten Sinusknotenzellen zur *in vitro* Erzeugung zellbasierter biologischer Herz-Schrittmacher.

## Claims

1. Method of producing sinoatrial node cells from stem cells, especially pluripotent stem cells, in which a nucleic acid is introduced into stem cells, as a result of which these express a TBX3 transcription factor, or a TBX3 protein is introduced into the stem cells,
**characterized in that**
a construct for expression of an antibiotic resistance gene which is controlled by an alpha-MHC (MYH6) promoter is additionally introduced and the resulting stem cells are differentiated in the presence of the antibiotic.

2. Method of producing sinoatrial node cells from stem cells according to Claim 1, **characterized in that** multipotent or pluripotent, preferably pluripotent, stem cells are used.

3. Method of producing sinoatrial node cells from stem cells according to Claim 1 or 2, **characterized in that** nonhuman embryonic stem cells or nonhuman induced stem cells or human induced stem cells or parthenogenetic stem cells or spermatogonial stem cells are used, preferably nonhuman embryonic stem cells or nonhuman induced stem cells or human induced stem cells.

4. Method of producing sinoatrial node cells from stem cells according to one or more of Claims 1-3, **characterized in that** the nucleic acid is selected from TBX3 DNA, especially TBX3 cDNA; TBX3 RNA, especially TBX3 mRNA.

5. Method of producing sinoatrial node cells from stem cells according to one or more of Claims 1-4, **characterized in that** the introduction of the TBX3 nucleic acid is effected by means of a vector, especially by means of an (over-)expression vector.

6. Method of producing sinoatrial node cells from stem cells according to one or more of Claims 1-5, **characterized in that** the antibiotic resistance gene is selected from aminoglycoside antibiotic resistance gene, more preferably from neomycin and puromycin resistance gene, most preferably new mice in resistance gene; and **in that** the antibiotic is correspondingly selected from aminoglycoside antibiotic, especially from neomycin and puromycin.

7. Use of sinoatrial node cells produced *in vitro* from stem cells according to a method according to one or more of Claims 1-6 for *in vitro* evaluation of medicaments.

8. Use of sinoatrial node cells produced *in vitro* from stem cells according to a method according to one or more of Claims 1-6 for *in vitro* construction of cardiac tissue and/or growing of cardiac tissue.

9. Use of sinoatrial node cells produced *in vitro* from stem cells according to a method according to one or more of Claims 1-6 for *in vitro* production of cell-based biological cardiac pacemakers.

## Revendications

1. Procédé de génération de cellules de noeuds sinusaux à partir de cellules souches, notamment de cellules souches pluripotentes, selon lequel un acide nucléique est introduit dans des cellules souches, ce qui amène celles-ci à exprimer un facteur de transcription TBX3, ou une protéine TBX3 est introduite dans les cellules souches,
**caractérisé en ce que**
une construction pour l'expression d'un gène de résistance à un antibiotique, qui est contrôlée par un promoteur alpha-MHC(MYH6), est en outre introduite et les cellules souches résultantes sont différenciées en présence de l'antibiotique.

2. Procédé de génération de cellules de noeuds sinusaux à partir de cellules souches selon la revendication 1, **caractérisé en ce que** des cellules souches multipotentes ou pluripotentes, de préférence pluripotentes, sont utilisées.

3. Procédé de génération de cellules de noeuds sinusaux à partir de cellules souches selon la revendication 1 ou 2, **caractérisé en ce que** des cellules souches embryonnaires non humaines ou des cellules souches induites non humaines ou des cellules souches induites humaines ou des cellules souches parthénogénétiques ou des cellules souches spermatogoniales sont utilisées, de préférence des cellules souches embryonnaires non humaines ou des cellules souches induites non humaines ou des cellules souches induites humaines.

4. Procédé de génération de cellules de noeuds sinusaux à partir de cellules souches selon une ou plusieurs des revendications 1 à 3, **caractérisé en ce que** l'acide nucléique est choisi parmi TBX3-DNS, notamment TBX3-cDNS ; TBX3-RNS, notamment TBX3-mRNS.

5. Procédé de génération de cellules de noeuds sinusaux à partir de cellules souches selon une ou plusieurs des revendications 1 à 4, **caractérisé en ce que** l'introduction de l'acide nucléique TBX3 a lieu au moyen d'un vecteur, notamment au moyen d'un vecteur de (sur)expression.

6. Procédé de génération de cellules de noeuds sinusaux à partir de cellules souches selon une ou plusieurs des revendications 1 à 5, **caractérisé en ce que** le gène de résistance à un antibiotique est choisi parmi un gène de résistance à un antibiotique aminoglycoside, de manière davantage préférée le gène de résistance à la néomycine et la puromycine, de manière préférée entre toutes le gène de résistance à la néomycine ; et **en ce que** l'antibiotique est choisi de manière correspondante parmi les antibiotiques aminoglycosides, notamment parmi la néomycine et la puromycine.

7. Utilisation de cellules de noeuds sinusaux générées *in vitro* à partir de cellules souches par un procédé selon une ou plusieurs des revendications 1 à 6 pour l'évaluation *in vitro* de médicaments.

8. Utilisation de cellules de noeuds sinusaux générées *in vitro* à partir de cellules souches par un procédé selon une ou plusieurs des revendications 1 à 6 pour la construction de tissus cardiaques et/ou la culture de tissus cardiaques *in vitro.*

9. Utilisation de cellules de noeuds sinusaux générées *in vitro* à partir de cellules souches par un procédé selon une ou plusieurs des revendications 1 à 6 pour la génération *in vitro* de stimulateurs cardiaques biologiques cellulaires.
